# EUROPÄISCHE PATENTANMELDUNG

(11) **EP 3 360 872 A1**
(43) Veröffentlichungstag der Anmeldung: **15.08.2018**
(21) Anmeldenummer: 17155823.2
(22) Anmeldetag: 13.02.2017
(51) Int. Cl.: C07D 405/04, C07D 417/04, A01N 43/54

(54) **UNSUBSTITUIERTE BENZYL-4-AMINOPICOLINSÄUREESTER UND PYRIMIDIN-4-CARBONSÄUREESTER, VERFAHREN ZU DEREN HERSTELLUNG SOWIE DEREN VERWENDUNG ALS HERBIZIDE UND PFLANZENWACHSTUMSREGULATOREN**

(71) Anmelder: Bayer CropScience Aktiengesellschaft, 40789 Monheim am Rhein (DE)
(72) Erfinder: HOFFMANN, Michael Gerhard, 78467 Konstanz (DE); DÖLLER, Uwe, 63110 Rodgau (DE); UENO, Chieko, Oyama-city Tohigi, 323-0807 (JP); DIETRICH, Hansjörg, 65835 Liederbach (DE); ROSINGER, Christopher Hugh, 65719 Hofheim (DE); MACHETTIRA, Anu Bheemaiah, 60326 Frankfurt (DE)
(74) Vertreter: BIP Patents

(57) **Zusammenfassung**

Herbizid wirksame unsubstituierte Benzyl-4-aminopicolinsäureester und Pyrimidin-4-carbonsäureester

Es werden Benzylcarbonsäurederivate von Benzoheterocyclylpyridinen und Benzoheterocyclylpyrimidinen der allgemeinen Formel (I) und ihre Verwendung als Herbizide beschrieben 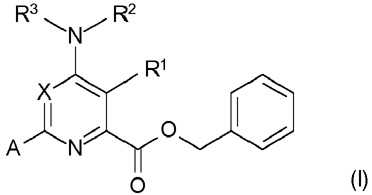 wobei X für N, CH oder CF und A für einen benzokondensierten Heterocyclus steht.

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus verschiedenen Schriften ist bekannt, dass substituierte Picolinsäurederivate und Pyrimidin-4-carbonsäurederivate herbizide Eigenschaften aufweisen: In WO 2003/011853 A1 werden polysubstituierte 6-Phenylpicolinsäurederivate mit herbizider Wirkung beschrieben. WO 2009/029735 A1 und WO 2010/125332 A1 beschreiben herbizide Wirkungen für polysubstituierte 2-Phenyl-4-pyrimidincarbonsäurederivate. Heteroaromatisch-substituierte Picolin- und Pyrimidincarbonsäuren mit herbiziden Eigenschaften werden in WO 2009/138712 A2 offenbart. Benzheteroaromatisch-substituierte Picolin- und 4-Pyrimidincarbonsäuren werden in WO 2013/014165 als Herbizide beansprucht. In WO 2007/080382 A1 und WO 2009/007751 A2 werden heteroaromatisch-substituierte Picolin- und Pyrimidincarbonsäuren mit pharmakologischen Wirkungen beschrieben.

Die dort beschriebenen Verbindungen zeigen jedoch häufig eine nicht ausreichende herbizide Wirksamkeit und/oder eine nicht ausreichende Selektivität in Nutzpflanzenkulturen.

Es wurden unsubstituierte Benzyl-4-aminopicolinsäureester und Pyrimidin-4-carbonsäureester gefunden, die besonders gut als Herbizide geeignet sind.

Ein Gegenstand der vorliegenden Erfindung sind Benzylpicolinsäure- und Pyrimidin-4-carbonsäureester der allgemeinen Formel (I) deren N-Oxide oder deren agrochemisch verträglichen Salze 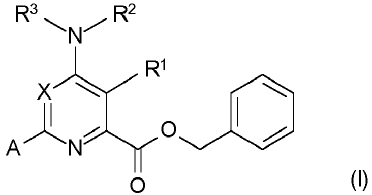
- A: bedeutet einen Rest der Gruppe bestehend aus A1 bis A20,

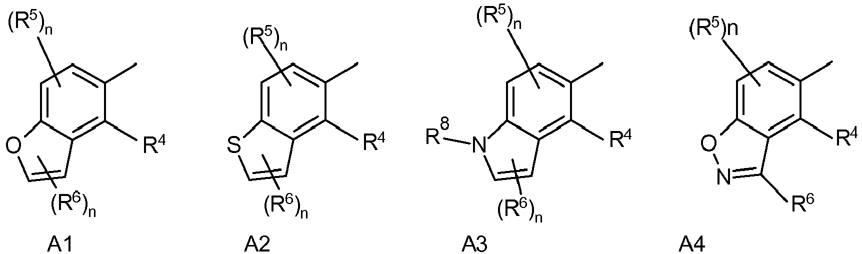

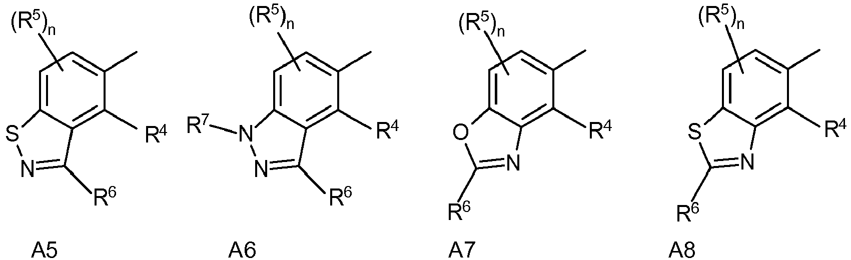

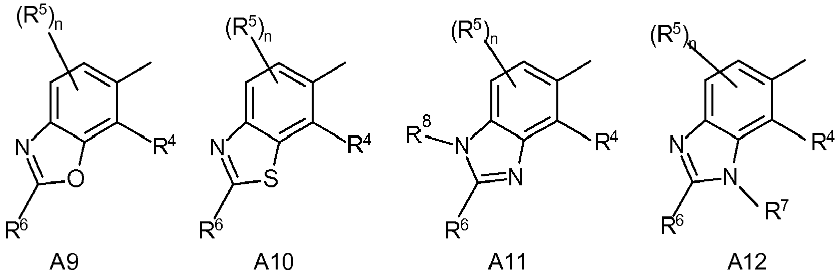

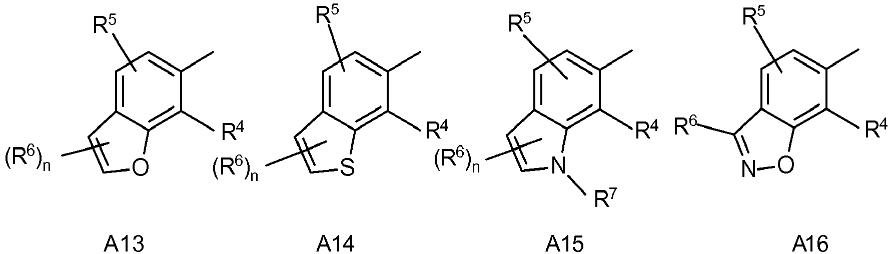

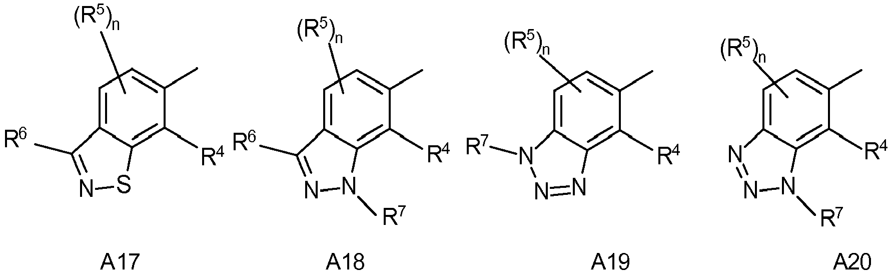

- R¹: bedeutet Chlor, Fluor oder Wasserstoff,
- R²: bedeutet Wasserstoff
- R³: bedeutet Wasserstoff,
- R⁴: bedeutet Wasserstoff, Halogen, OH, NH₂, CN, (C₁-C₃)-Alkyl, (C₁-C₃)-Alkoxy, (C₁-C₃)-Alkylamino oder Cyclopropyl,
- R⁵: bedeutet Wasserstoff, Halogen, OH, NH₂, CN, (C₁-C₃)-Alkyl, (C₁-C₃)-Alkoxy, Cyclopropyl oder Vinyl,
- R⁶: bedeutet Wasserstoff, Halogen, (C₁-C₃)-Alkyl, (C₁-C₃)-Alkoxy, (C₁-C₃)-Alkylthio, Cyclopropyl, (C₁-C₃)-Alkylamino oder Phenyl,
- R⁷: bedeutet Wasserstoff, (C₁-C₆)-Alkyl, (C₁-C₄)-Alkylcarbonyl, (C₁-C₆)-Alkoxycarbonyl, oder Phenyl,
- X: bedeutet N, CH oder CF, und
- n: bedeutet 0, 1 oder 2.

Eine erste Ausführungsform der vorliegenden Erfingung umfasst Verbindungen der allgemeinen Formel (I), in welchen
- A: bevorzugt ausgewählt ist aus der Gruppe bestehend aus A1 bis A3, A7 bis A15 und A17 bis A18,

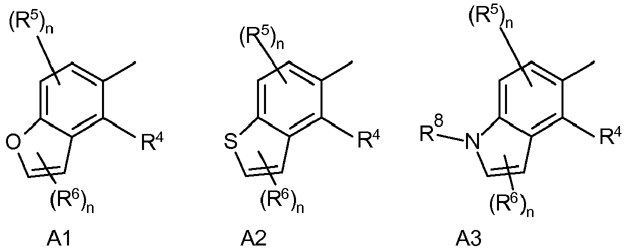

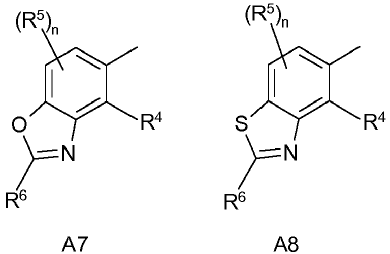

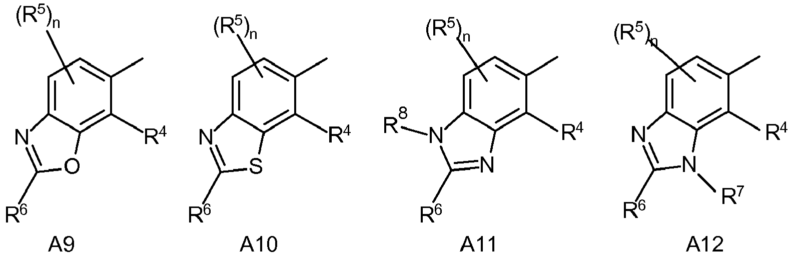

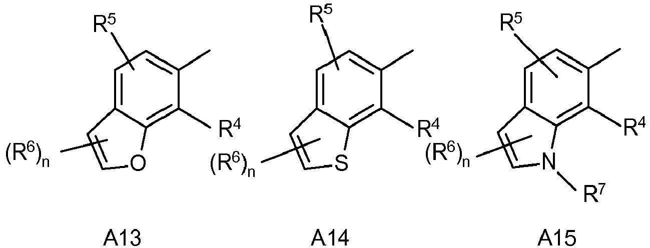

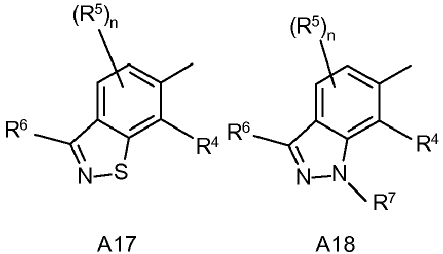

- A: ist besonders bevorzugt ausgewählt aus der Gruppe bestehend aus den Resten A1 bis A3 und A13 bis A15;
- A: ist am meisten bevorzugt A2 oder A15.

Eine zweite Ausführungsform der vorliegenden Erfingung umfasst Verbindungen der allgemeinen Formel (I), in welchen
- R¹: bevorzugt Chlor bedeutet.

Eine dritte Ausführungsform der vorliegenden Erfingung umfasst Verbindungen der allgemeinen Formel (I), in welchen
- R²: bevorzugt Wasserstoff bedeutet.

Eine vierte Ausführungsform der vorliegenden Erfingung umfasst Verbindungen der allgemeinen Formel (I), in welchen
- R³: bevorzugt Wasserstoff bedeutet.

Eine fünfte Ausführungsform der vorliegenden Erfingung umfasst Verbindungen der allgemeinen Formel (I), in welchen
- R⁴: bevorzugt Wasserstoff oder Halogen, und besonders bevorzugt Wasserstoff und Fluor bedeutet.

Eine sechste Ausführungsform der vorliegenden Erfingung umfasst Verbindungen der allgemeinen Formel (I), in welchen
- R⁵: bevorzugt Wasserstoff oder Halogen, und besonders bevorzugt Wasserstoff bedeutet.

Eine siebte Ausführungsform der vorliegenden Erfingung umfasst Verbindungen der allgemeinen Formel (I), in welchen
R⁶ bevorzugt Wasserstoff, Halogen oder (C₁-C₃)-Alkyl, und besonders bevorzugt Wasserstoff bedeutet.

Eine achte Ausführungsform der vorliegenden Erfingung umfasst Verbindungen der allgemeinen Formel (I), in welchen
R⁷ bevorzugt Wasserstoff, (C₁-C₄)-Alkyl, (C₁-C₄)-Alkylcarbonyl oder (C₁-C₄)-Alkoxycarbonyl, besonders bevorzugt Wasserstoff, (C₁-C₃)-Alkyl, (C₁-C₄)-Alkylcarbonyl oder (C₁-C₄)-Alkoxycarbonyl, und am meisten bevorzugt Wasserstoff bedeutet.

Eine neunte Ausführungsform der vorliegenden Erfingung umfasst Verbindungen der allgemeinen Formel (I), in welchen
- X: bevorzugt CH oder CF bedeuten.

Eine zehnte Ausführungsform der vorliegenden Erfingung umfasst Verbindungen der allgemeinen Formel (I), in welchen
- n: bevorzugt 0 oder 1, und besonders bevorzugt 0 bedeutet.

Im Rahmen der vorliegenden Erfindung ist es möglich, die einzelnen bevorzugten, besonders bevorzugten und ganz besonders bevorzugten Bedeutungen für die Substituenten A, R¹ bis R⁷ und X beliebig miteinander zu kombinieren, wobei die Laufzahl n 0, 1 oder 2 ist, bevorzugt 0 oder 1, und ganz besonders bevorzugt 0 ist.

Das heißt, dass Verbindungen der allgemeinen Formel (I) von der vorliegenden Erfindung umfasst sind, in welchen beispielsweise der Rest A eine bevorzugte Bedeutung aufweist und die Substituenten R¹ bis R⁷ die allgemeine Bedeutung aufweisen oder aber der Substituent R² eine bevorzugte Bedeutung aufweist, der Substituent R⁴ eine besonders bevorzugte Bedeutung aufweist und die übrigen Substituenten eine allgemeine Bedeutung aufweisen.

Drei dieser Kombinationen der oben für die Substituenten A, R¹ bis R⁷ und X, sowie für die Laufzahlen n und m gegebenen Definitionen werden nachfolgend beispielhaft erläutert und jeweils als weitere Ausführungsformen aus Gründen der Klarheit explizit offenbart:
Eine elfte Ausführungsform der vorliegenden Erfingung umfasst Verbindungen der allgemeinen Formel (I), deren N-Oxide oder deren agrochemisch verträglichen Salze, in welchen
   - A: ausgewählt ist aus A1 bis A3 und A13 bis A15,
   - R¹: Chlor bedeutet,
   - R²: Wasserstoff bedeutet,
   - R³: Wasserstoff bedeutet,
   - R⁴: Wasserstoff oder Halogen bedeutet,
   - R⁵: Wasserstoff oder Halogen bedeutet,
   - R⁶: Wasserstoff, Halogen oder (C₁-C₃)-Alkyl bedeutet,
   - R⁷: Wasserstoff, (C₁-C₄)-Alkyl, (C₁-C₄)-Alkylcarbonyl oder (C₁-C₄)-Alkoxycarbonyl bedeutet,
   - X: N, CH oder CF bedeutet, und
   - n: 0 oder 1 bedeutet.

Eine zwölfte Ausführungsform der vorliegenden Erfingung umfasst Verbindungen der allgemeinen Formel (I), deren N-Oxide oder deren agrochemisch verträglichen Salze, in welchen
- A: ausgewählt ist aus A1 bis A3 und A13 bis A15,
- R¹: Chlor bedeutet,
- R²: Wasserstoff bedeutet,
- R³: Wasserstoff bedeutet,
- R⁴: Wasserstoff oder Fluor bedeutet,
- R⁵: Wasserstoff bedeutet,
- R⁶: Wasserstoff bedeutet,
- R⁷: Wasserstoff, (C₁-C₃)-Alkyl, (C₁-C₄)-Alkylcarbonyl oder (C₁-C₄)-Alkoxycarbonyl bedeutet,
- X: CH oder CF bedeutet, und
- n: 0 bedeutet.

Eine dreizehnte Ausführungsform der vorliegenden Erfingung umfasst Verbindungen der allgemeinen Formel (I), deren N-Oxide oder deren agrochemisch verträglichen Salze, in welchen
- A: A2 oder A15 bedeutet,
- R¹: Chlor bedeutet,
- R²: Wasserstoff bedeutet,
- R³: Wasserstoff bedeutet,
- R⁴: Wasserstoff oder Fluor bedeutet,
- R⁵: Wasserstoff bedeutet,
- R⁶: Wasserstoff bedeutet,
- R⁷: Wasserstoff, (C₁-C₃)-Alkyl, (C₁-C₄)-Alkylcarbonyl oder (C₁-C₄)-Alkoxycarbonyl bedeutet,
- X: CH oder CF bedeutet, und
- n: 0 bedeutet.

Alkylcarbonyl (Alkyl-C(=O)-) bedeutet gesättigte, geradkettige oder verzweigte Alkylreste, die über -C(=O)- an das Gerüst gebunden sind, wie (C₁-C₁₀)-, (C₁-C₆)- oder (C₁-C₄)-Alkylcarbonyl. Die Anzahl der C-Atome bezieht sich dabei auf den Alkylrest in der Alkylcarbonylgruppe.

Alkyl bedeutet gesättigte, geradkettige oder verzweigte Kohlenwasserstoffreste mit 1 bis 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Halogenalkyl bedeutet geradkettige oder verzweigte Alkylgruppen mit 1 bis 8 Kohlenstoffatomen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Alkenyl bedeutet ungesättigte, geradkettige oder verzweigte Kohlenwasserstoffreste mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkinyl bedeutet geradkettige oder verzweigte Kohlenwasserstoffreste mit 2 bis 8 Kohlenstoffatomen und einer Dreifachbindung in einer beliebigen Position, z.B. C₂-C₆-Alkinyl wie Ethinyl, 1-Propinyl, 2-Propinyl (oder Propargyl), 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Alkoxy bedeutet gesättigte, geradkettige oder verzweigte Alkoxyreste mit 1 bis 8 Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
Halogenalkoxy bedeutet geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluorethoxy und 1,1,1-Trifluorprop-2-oxy.

Alkylthio bedeutet gesättigte, geradkettige oder verzweigte Alkylthioreste mit 1 bis 8 Kohlenstoffatomen, z.B. C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methylpentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
Halogenalkylthio bedeutet geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio.

Aryl bedeutet Phenyl oder Naphthyl.

Die Verbindungen der Formel (I) können, auch in Abhängigkeit von der Art der Substituenten, als geometrische und/oder optische Isomere oder Isomeren-gemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Ein Metallionäquivalent bedeutet ein Metallion mit einer positiven Ladung wie Na⁺, K⁺, (Mg²⁺)_{1/2}, (Ca²⁺)_{1/2}, MgH⁺, CaH⁺, (Al³⁺)_{1/3} (Fe²⁺)_{1/2} oder (Fe³⁺)_{1/3}.

Halogen bedeutet Fluor, Chlor, Brom und Jod.

Ist eine Gruppe mehrfach durch Reste substituiert, so ist darunter zu verstehen, daß diese Gruppe durch ein oder mehrere gleiche oder verschiedene der genannten Reste substituiert ist.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel (I) Amino, Alkylamino oder andere, basische Eigenschaften induzierende Gruppen, so können diese Verbindungen mit Säuren zu Salzen umgesetzt werden oder fallen durch die Synthese direkt als Salze an.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Die Verbindungen der allgemeinen Formel (I) können je nach Art und Verknüpfung der Substituenten als Stereoisomere vorliegen. Sind beispielsweise ein oder mehrere asymmetrisch substiuierte Kohlenstoffatome oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

In allen nachfolgend genannten Formeln haben die Substituenten und Symbole, sofern nicht anders definiert, dieselbe Bedeutung wie unter Formel (I) beschrieben.

Verbindungen der Formel (I), können beispielsweise gemäß nachfolgendem Schema durch basenkatalysierteVeresterung einer Carbonsäure der Formel II mit einem Benzylbromid der Formel III hergestellt werden. In diesem Schema steht Het für die an den Phenylring ankondensierten Heterocyclen der Gruppen A1 bis A20.

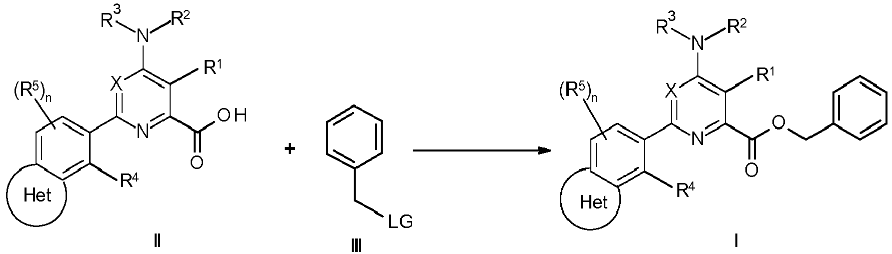

Die Carbonsäuren der Formel (II) sind beispielsweise aus WO2013/14165 A1 bekannt, oder können nach dem Fachmann an sich bekannten Methoden hergestellt werden. Die Benzyl-Derivate der Formel (III) sind kommerziell erhältlich, oder können nach dem Fachmann an sich bekannten Methoden hergestellt werden.

Bevorzugt sind die in der folgenden Tabelle 1 genannten Verbindungen der Formel (I), 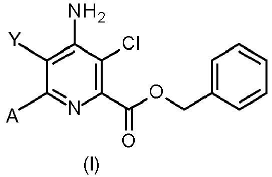 mit X = C-Y, R¹ = Cl, R² = R³ = H

**Tabelle 1**

| **Nummer** | **A** | **Y** |
|---|---|---|
| I-01 | 1-Benzothiophen-6-yl | H |
| I-02 | 1-Benzofuran-6-yl | F |
| I-03 | 1-Benzofuran-5-yl | F |
| I-04 | 1-Benzothiophen-5-yl | H |
| I-05 | 1-Benzofuran-6-yl | H |
| I-06 | 1-Benzothiophen-5-yl | F |
| I-07 | (7-Fluor-1-benzofuran-6-yl) | H |
| I-08 | 1-Benzofuran-5-yl | H |
| I-09 | (6-Fluor-1-benzothiophen-5-yl) | H |
| I-10 | 1-Benzothiophen-6-yl | F |
| I-11 | 1,3-Benzothiazol-6-yl | F |
| I-12 | 1,3-Benzothiazol-6-yl | H |
| I-13 | (4,7-Difluor-1-benzofuran-5-yl) | H |
| I-14 | (6-Fluor-1-benzofuran-5-yl) | F |

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry-Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und A. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum^{™} GAT^{™} (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sei. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum^{™} GAT^{™} (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Die Safener sind vorzugsweise ausgewählt aus der Gruppe bestehend aus:
S1) Verbindungen der Formel (S1), 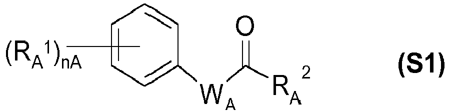 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl;
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴),

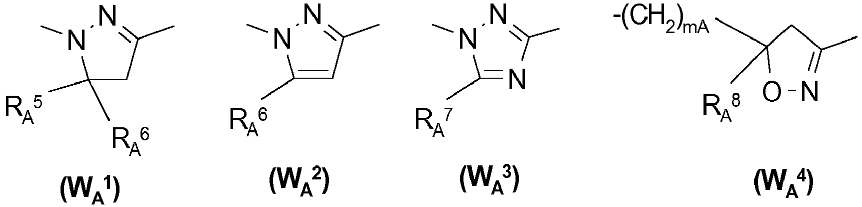

   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   - R_{A}⁴: ist Wasserstoff, (C₁-C₆)Alkyl, (C₁-C₆)Alkoxy oder substituiertes oder unsubstituiertes Phenyl;
   - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1°), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-3-carbonsäure (S1-10) oder 5,5-Diphenyl-2-isoxazolin-3-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 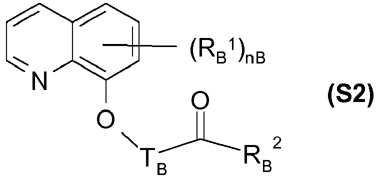 wobei die Symbole und Indizes folgende Bedeutungen haben:
   - R_{B}¹: ist Halogen, (C₁-C₄)Alkyl, (C₁-C₄)Alkoxy, Nitro oder (C₁-C₄)Haloalkyl;
   - n_{B}: ist eine natürliche Zahl von 0 bis 5, vorzugsweise 0 bis 3;
   - R_{B}²: ist OR_{B}³, SR_{B}³ oder NR_{B}³R_{B}⁴ oder ein gesättigter
   oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 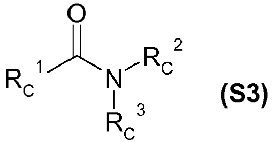 wobei die Symbole und Indizes folgende Bedeutungen haben:
   - R_{C}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{C}², R_{C}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl,(C₁-C₄)Alkoxy-(C₁-C₄)alkyl,Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4),
   "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9)
   ((RS)-1-Dichloracetyl-3,3,8a-trimethylperhydropyrrolo[1,2-a]pyrimidin-6-on) der Firma BASF, "Furilazol" oder "MON 13900" ((RS)-3-Dichloracetyl-5-(2-furyl)-2,2-dimethyloxazolidin)
   (S3-10); sowie dessen (R)-Isomer (S3-11).
S4) N-Acylsulfonamide der Formel (S4) und ihre Salze, 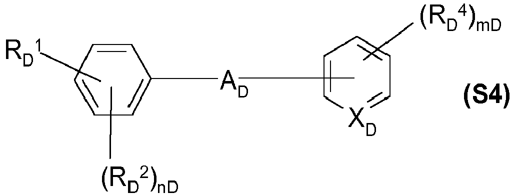 worin die Symbole und Indizes folgende Bedeutungen haben:
   - A_{D}: ist SO₂-NR_{D}³-CO oder CO-NR_{D}³-SO₂;
   - X_{D}: ist CH oder N;
   - R_{D}¹: ist CO-NR_{D}⁵R_{D}⁶ oder NHCO-R_{D}⁷;
   - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   - R_{D}³: ist Wasserstoff, (C₁-C₄)Alkyl, (C₂-C₄)Alkenyl oder (C₂-C₄)Alkinyl;
   - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   - R_{D}⁶: ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
   - R_{D}⁵ und R_{D}⁶: gemeinsam mit dem dem sie tragenden Stickstoffatom einen Pyrrolidinyl- oder Piperidinyl-Rest bilden;
   - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - n_{D}: ist 0, 1 oder 2;
   - m_{D}: ist 1 oder 2;
   - v_{D}: ist 0, 1, 2 oder 3;
   davon bevorzugt sind Verbindungen vom Typ der N-Acylsulfonamide, z.B. der nachfolgenden Formel (S4^{a}), die z. B. bekannt sind aus WO-A-97/45016 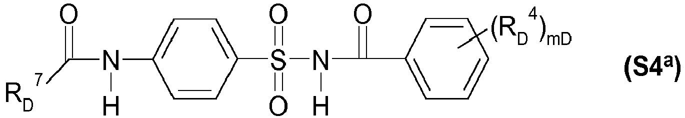 worin
   R_{D}⁷ (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   R_{D}⁴ Halogen, (C₁-C₄)Alkyl, (C₁-C₄)Alkoxy, CF_{3;}
   m_{D} 1 oder 2;
   v_{D} ist 0, 1, 2 oder 3 bedeutet;
   sowie
   Acylsulfamoylbenzoesäureamide, z.B. der nachfolgenden Formel (S4^{b}), die z.B. bekannt sind aus WO-A-99/16744, 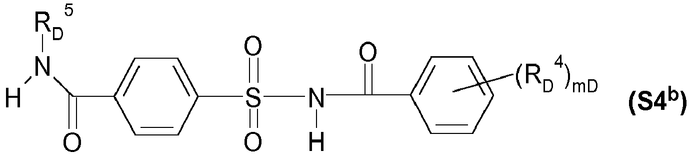
   z.B. solche worin
   R_{D}⁵ = Cyclopropyl und (R_{D}⁴) = 2-OMe ist("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5);
   sowie
   Verbindungen vom Typ der N-Acylsulfamoylphenylharnstoffe der Formel (S4^{c}), die z.B. bekannt sind aus der EP-A-365484, 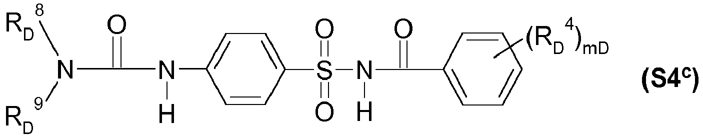 worin
   R_{D}⁸ und R_{D}⁹ unabhängig voneinander Wasserstoff, (C₁-C₈)Alkyl, (C₃-C₈)Cycloalkyl, (C₃-C₆)Alkenyl, (C₃-C₆)Alkinyl,
   R_{D}⁴ Halogen, (C₁-C₄)Alkyl, (C₁-C₄)Alkoxy, CF_{3;}
   m_{D} 1 oder 2 bedeutet;
   beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   sowie
   N-Phenylsulfonylterephthalamide der Formel (S4^{d}), die z.B. bekannt sind aus CN 101838227, 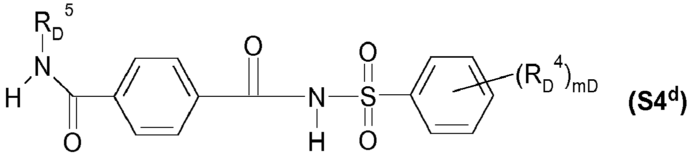 z.B. solche worin
   - R_{D}⁴: Halogen, (C₁-C₄)Alkyl, (C₁-C₄)Alkoxy, CF₃;
   - m_{D}: 1 oder 2;
   - R_{D}⁵: Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.
S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7), wie sie in der WO-A-1998/38856 beschrieben sind 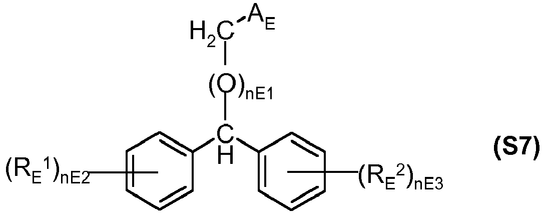 worin die Symbole und Indizes folgende Bedeutungen haben:
   - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{E}: ist COOR_{E}³ oder COSR_{E}⁴;
   - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{E}¹: ist 0 oder 1,
   - n_{E}², n_{E}³: sind unabhängig voneinander 0, 1 oder 2,
   vorzugsweise:
   Diphenylmethoxyessigsäure,
   Diphenylmethoxyessigsäureethylester,
   Diphenylmethoxyessigsäuremethylester (CAS-Reg.Nr. 41858-19-9) (S7-1).
S8) Verbindungen der Formel (S8), wie sie in der WO-A-98/27049 beschrieben sind 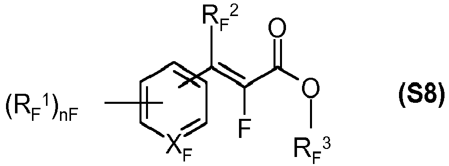 worin
   - X_{F}: CH oder N,
   - n_{F}: für den Fall, dass X_{F}=N ist, eine ganze Zahl von 0 bis 4, und
   für den Fall, dass X_{F}=CH ist, eine ganze Zahl von 0 bis 5,
   - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   - R_{f}²: Wasserstoff oder (C₁-C₄)Alkyl,
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; bedeuten, oder deren Salze,
   vorzugsweise Verbindungen worin
   - X_{F}: CH,
   - n_{F}: eine ganze Zahl von 0 bis 2,
   - R_{F}¹: Halogen, (C₁-C₄)Alkyl, (C₁-C₄)Haloalkyl, (C₁-C₄)Alkoxy, (C₁-C₄)Haloalkoxy,
   - R_{F}²: Wasserstoff oder (C₁-C₄)Alkyl,
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten,
   oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 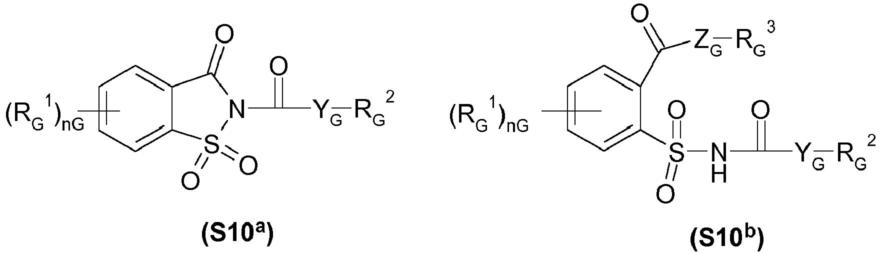 worin
   - R_{G}¹: Halogen, (C₁-C₄)Alkyl, Methoxy, Nitro, Cyano, CF₃, OCF₃,
   - Y_{G}, Z_{G}: unabhängig voneinander O oder S,
   - n_{G}: eine ganze Zahl von 0 bis 4,
   - R_{G}²: (C₁-C₁₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S 11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG 838" (CAS-Reg.Nr. 133993-74-5) (2-propenyl 1-oxa-4-azaspiro[4.5]decan-4-carbodithioat) (S13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorothioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY 93" (*S*-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC 940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere 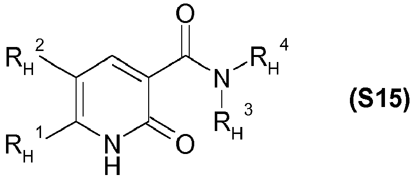 wie sie in der WO-A-2008/131861 und WO-A-2008/131860 beschrieben sind
   worin
   - R_{H}¹: einen (C₁-C₆)Haloalkylrest bedeutet, und
   - R_{H}²: Wasserstoff oder Halogen bedeutet, und
   - R_{H}³, R_{H}⁴: unabhängig voneinander Wasserstoff, (C₁-C₁₆)Alkyl, (C₂-C₁₆)Alkenyl oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-_{C4})Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet oder
   - R_{H}³: (C₁-C₄)-Alkoxy, (C₂-C₄)Alkenyloxy, (C₂-C₆)Alkinyloxy oder (C₂-C₄)Haloalkoxy bedeutet, und
   - R_{H}⁴: Wasserstoff oder (C₁-C₄)-Alkyl bedeutet, oder
   - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Bevorzugte Safener sind: Cloquintocet-mexyl, Cyprosulfamid, Fenchlorazol-ethylester, Isoxadifen-ethyl, Mefenpyr-diethyl, Fenclorim, Cumyluron, S4-1 und S4-5, besonders bevorzugt sind: Cloquintocet-mexyl, Cyprosulfamid, Isoxadifen-ethyl und Mefenpyr-diethyl.

Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoffe. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2009 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:

Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlorpotassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenacsodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, - dimethylammonium, -diolamin, -ethyl, 2-ethylhexyl, -isobutyl, -isooctyl, -isopropylammonium, - potassium, -trüsopropanolammonium und -trolamine, 2,4-DB, 2,4-DB-butyl, -dimethylammonium, isooctyl, -potassium und -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyrsodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, - dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, - potassium, -sodium und -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuronmethyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, imazamethabenz, Imazamethabenz-methyl, imazamox, imazamox-ammonium, imazapic, imazapic-ammonium, imazapyr, imazapyr-isopropylammonium, imazaquin, imazaquinammonium, imazethapyr, imazethapyr-immonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, - isopropylammonium, -potassium und -sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuronethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, , SYN-523, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazonemethyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, sowie die folgenden Verbindungen:

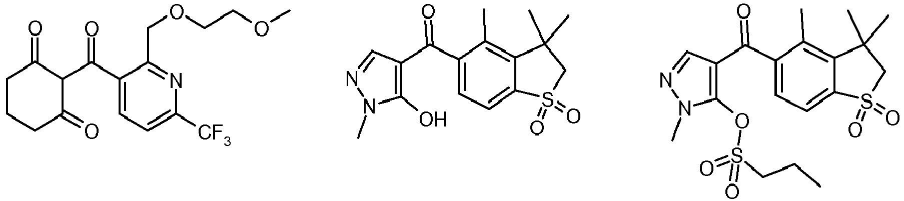

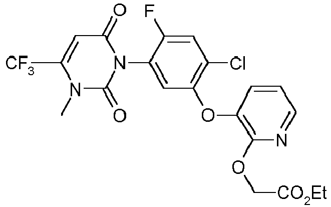

Beispiele für Pflanzenwachstumsregulatoren als mögliche Mischungspartner sind:
Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothaldipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2-naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung näher.

### A. Chemische Beispiele

### 1. Herstellung von Benzyl-4-amino-6-(1-benzothiophen-5-yl)-3-chlor-5-fluorpyridin-2-carboxylat (Beispiel Nr. I-06)

Zu einer Lösung von 0,14g (0,46mmol) 4-Amino-6-(1-benzothiophen-5-yl)-3-chlor-5-fluorpyridin-2-carbonsäure in 10ml THF gibt man nacheinander 0,13ml Triethylamin und 0,18g (0,69mmol) Benzylbromid, und rührt diese Reaktionsmischung 12 Stunden bei Raumtemperatur. Zur Aufarbeitung wird bis zur Trockne eingeengt und das so erhaltene Rohgemisch säulenchromatographisch über Kieselgel (Heptan/Ethylacetat 2/8) gereinigt. Man erhält 0,049g (27%) Produkt als farbloses Öl.

### 2. Herstellung von Benzyl-4-amino-3-chlor-6-(7-fluor-1-benzofuran-6-yl)pyridin-2-carboxylat (Beispiel Nr. I-07)

Zu einer Lösung von 0,08g (0,25mmol) 4-Amino-3-chlor-6-(7-fluor-1-benzofuran-6-yl)pyridin-2-carbonsäure in 10ml THF gibt man nacheinander 0,07ml Triethylamin und 0,064g (0,37mmol) Benzylbromid, und rührt diese Reaktionsmischung 12 Stunden bei Raumtemperatur. Zur Aufarbeitung wird bis zur Trockne eingeengt und das so erhaltene Rohgemisch säulenchromatographisch über Kieselgel (Heptan/Ethylacetat 2/8) gereinigt. Man erhält 0,06g (58%) Produkt als farbloses Öl.

### NMR-Daten ausgewählter Beispiele

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| Beispiel 1-01: ¹H-NMR(400.0 MHz, CDCl3): |
| δ= 8.498(6.6);8.496(7.0);8.494(6.5);7.896(3.2);7.892(3.0);7.875(8.2);7.871(8.2);7.849(9.0);7.848(9.0);7 .828(3.4);7.604(1.6);7.600(1.7);7.586(3.3);7.581(3.3);7.567(1.7);7.563(1.8);7.501(12.2);7.488(14.5);7.3 65(1.1);7.360(1.1);7.351(1.4);7.345(8.8);7.343(8.2);7.332(7.5);7.330(7.1);7.326(2.4);7.321(1.5);7.312(1 .4);7.308(1.4);7.264(0.6);7.263(0.7);7.262(0.8);7.261(1.0);7.256(63.5);7.252(0.6);7.192(2.9);7.189(3.2); 7.173(4.9);7.170(5.3);7.159(19.8);7.154(2.5);7.151(2.4);7.122(2.7);7.119(2.4);7.101(2.3);7.097(3.6);7.0 94(2.5);7.076(2.1);7.073(2.0);5.543(16.0);5.294(8.7);4.779(6.3);1.565(1.6);0.008(0.8);0.000(28.9);-0.008(0.8) |
| Beispiel I-02: ¹H-NMR(400.0 MHz, CDCl3): |
| δ= 8.110(2.9);8.107(4.2);8.106(5.6);8.104(4.2);8.102(2.8);7.833(4.3);7.829(4.1);7.812(5.2);7.809(5.2);7 .678(8.7);7.672(8.7);7.642(5.3);7.641(5.3);7.622(4.4);7.621(4.2);7.384(0.8);7.382(0.7);7.369(1.2);7.366 (1.1);7.364(2.1);7.349(2.5);7.343(1.6);7.329(2.0);7.280(3.1);7.278(2.4);7.262(4.9);7.260(5.3);7.258(53. 5);7.254(1.7);7.252(1.1);7.240(1.3);7.239(1.4);7.238(1.1);7.234(1.7);7.230(1.2);7.157(18.4);7.058(1.0); 7.056(1.0);7.052(0.9);7.049(0.9);7.037(1.5);7.034(1.7);7.030(1.4);7.028(1.5);7.016(0.8);7.013(0.8);7.01 0(0.8);7.007(0.8);6.788(5.5);6.785(5.7);6.782(5.6);6.780(5.3);5.452(16.0);5.296(0.8);4.788(4.1);1.549(1 0.2);0.008(0.7);0.000(22.6);-0.008(0.6) |
| Beispiel I-03: ¹H-NMR(400.0 MHz, CDCl3): |
| δ= 8.111(1.4);7.880(0.6);7.876(1.1);7.872(0.6);7.860(0.7);7.856(1.3);7.852(0.7);7.705(0.6);7.700(2.9);7 .695(2.9);7.692(0.8);7.673(1.8);7.652(1.4);7.442(2.4);7.437(1.1);7.425(0.8);7.420(2.6);7.308(0.7);7.286 (0.7);7.258(49.5);6.925(3.0);6.920(1.0);6.908(1.5);6.903(3.0);6.886(0.8);6.858(0.5);6.806(1.6);6.803(1. 6);6.800(1.6);6.798(1.5);6.661(0.6);5.384(6.1);4.852(1.4);4.632(0.6);4.617(0.6);3.817(16.0);3.812(4.7); 3.778(2.9);1.536(13.4);0.008(0.7);0.000(21.0);-0.008(0.6) |
| Beispiel I-04: ¹H-NMR(400.0 MHz, CDCl3): |
| δ= 8.196(0.9);8.115(4.4);7.969(0.7);7.949(0.9);7.882(2.0);7.878(3.5);7.874(2.0);7.861(2.4);7.857(4.3);7 .854(2.3);7.709(2.3);7.706(8.4);7.700(9.6);7.697(2.6);7.680(6.4);7.659(4.6);7.604(1.0);7.585(1.1);7.517 (1.0);7.499(3.7);7.494(1.6);7.486(3.9);7.477(4.2);7.469(1.7);7.464(4.1);7.409(1.2);7.356(1.0);7.343(1.1) ;7.334(1.3);7.320(1.3);7.309(0.7);7.258(157.8);7.209(1.1);7.155(1.0);7.151(1.0);7.135(1.0);7.132(1.0);7 .105(0.7);7.097(5.0);7.092(1.7);7.081(1.8);7.076(9.2);7.070(1.9);7.067(2.0);7.059(1.7);7.054(4.5);7.045 (2.8);7.039(0.6);7.023(1.2);6.994(0.9);6.929(0.7);6.915(0.8);6.907(1.0);6.893(1.1);6.828(1.2);6.826(1.2) ;6.822(1.8);6.816(1.6);6.814(1.8);6.811(5.4);6.808(5.6);6.806(5.9);6.803(5.2);6.785(1.9);6.763(0.8);5.4 08(16.0);5.004(1.2);4.872(4.2);4.675(1.8);4.661(1.8);4.380(1.0);1.623(0.6);1.609(1.2);1.594(0.6);1.533( 31.0);1.333(0.6);1.284(0.8);1.256(0.7);0.008(2.3);0.000(67.5);-0.008(2.3) |
| Beispiel I-05: ¹H-NMR(400.0 MHz, CDCl3): |
| δ= 8.166(5.1);8.162(5.2);7.853(3.2);7.848(3.1);7.831(3.7);7.827(3.6);7.647(6.9);7.641(6.9);7.542(4.5);7 .540(3.1);7.522(2.8);7.520(4.0);7.518(2.8);7.503(3.8);7.498(1.6);7.490(4.0);7.481(4.4);7.473(1.7);7.468 (4.1);7.258(66.9);7.122(16.1);7.101(0.6);7.093(5.2);7.088(1.6);7.077(1.9);7.072(9.7);7.066(1.7);7.055(1 .8);7.050(4.6);6.810(5.0);6.807(5.1);6.804(5.0);6.802(4.8);5.421(16.0);5.296(1.4);4.763(4.4);1.551(12.6 );0.008(0.8);0.000(25.2);-0.008(0.7) |
| Beispiel I-06: ¹H-NMR(400.0 MHz, CDCl3): |
| δ= 8.192(7.6);8.189(7.6);7.861(5.0);7.857(4.8);7.840(5.7);7.835(5.4);7.653(10.7);7.648(10.5);7.555(5.0) ;7.553(6.9);7.551(4.7);7.534(4.3);7.532(6.0);7.530(4.5);7.527(1.8);7.518(0.6);7.510(1.7);7.505(1.8);7.5 01(1.8);7.488(1.8);7.484(1.8);7.479(1.7);7.463(1.5);7.268(0.5);7.267(0.6);7.266(0.7);7.264(1.1);7.264(1 .3);7.259(87.5);7.255(0.8);7.254(0.6);7.209(0.6);7.164(0.5);7.151(27.6);7.005(2.2);6.995(0.7);6.989(2.3 );6.981(2.6);6.964(2.6);6.957(2.2);6.941(2.1);6.823(8.0);6.821(8.1);6.818(8.0);6.815(7.7);5.457(16.0);5. 297(11.7);4.795(6.5);1.552(9.8);1.255(0.6);0.008(1.2);0.000(37.8);-0.008(1.1) |
| Beispiel I-08: ¹H-NMR(400.0 MHz, CDCl3): |
| δ= 8.495(2.5);8.493(2.7);8.491(2.6);7.893(1.0);7.890(1.0);7.872(3.2);7.868(3.3);7.854(3.6);7.852(3.5);7 .833(1.1);7.832(1.0);7.507(4.8);7.494(5.6);7.350(2.7);7.348(2.7);7.337(2.3);7.335(2.2);7.258(26.6);7.24 4(0.7);7.230(0.7);7.225(0.8);7.224(0.8);7.223(0.8);7.211(0.8);7.208(1.0);7.204(0.8);7.189(0.8);7.171(7. 6);6.946(1.0);6.940(1.0);6.924(1.0);6.919(1.6);6.915(1.1);6.898(0.9);6.893(0.9);5.477(4.9);5.476(5.1);5. 474(4.9);4.791(2.5);4.017(8.5);4.014(16.0);4.012(8.4);1.559(6.1);0.000(11.7) |
| Beispiel I-09: ¹H-NMR(400.0 MHz, CDCl3): |
| δ= 8.411(4.8);8.409(4.6);7.931(1.7);7.910(7.2);7.899(5.0);7.895(4.7);7.878(1.2);7.874(1.2);7.474(4.9);7 .460(7.0);7.398(5.4);7.384(3.8);7.379(1.2);7.365(1.2);7.360(2.4);7.345(2.7);7.340(1.8);7.327(1.0);7.324 (1.6);7.280(2.0);7.275(3.9);7.273(4.2);7.257(45.7);7.184(14.8);7.059(0.9);7.057(1.0);7.053(0.9);7.050(0 .9);7.036(1.7);7.031(1.6);7.017(0.9);7.013(1.0);7.008(0.8);6.858(0.7);5.457(16.0);5.381(0.8);5.294(12.8 );4.797(4.4);1.561(1.2);0.008(0.5);0.000(15.8) |
| Beispiel I-10: ¹H-NMR(400.0 MHz, CDCl3): |
| δ= 8.509(2.8);8.507(4.8);8.505(5.3);8.503(5.0);8.501(2.7);7.908(2.4);7.904(2.3);7.887(6.1);7.883(6.1);7 .861(6.2);7.860(6.3);7.840(2.4);7.839(2.3);7.508(8.1);7.494(9.4);7.384(1.3);7.369(1.2);7.363(3.1);7.357 (0.5);7.351(5.6);7.349(7.2);7.343(2.4);7.338(4.1);7.336(4.1);7.329(2.0);7.278(3.1);7.276(2.6);7.272(1.8) ;7.268(2.0);7.266(2.1);7.262(3.4);7.256(37.5);7.248(1.4);7.248(1.5);7.242(1.7);7.238(1.1);7.179(16.4);7 .061(0.9);7.058(1.0);7.055(0.9);7.052(0.9);7.040(1.6);7.038(1.6);7.033(1.5);7.032(1.4);7.019(0.8);7.016(0.8);7.013(0.8);7.010(0.7);5.458(16.0);5.294(3.1);4.797(4.4);1.562(2.6);0.008(0.5);0.000(16.1) |
| Beispiel I-11: ¹H-NMR(400.0 MHz, CDCl3): |
| δ= 8.203(1.0);8.200(1.0);7.877(0.6);7.872(0.6);7.855(0.8);7.851(0.7);7.829(0.7);7.655(1.4);7.650(1.4);7 .558(0.7);7.556(0.9);7.554(0.6);7.536(0.9);7.535(1.0);7.533(0.6);7.518(0.9);7.259(49.0);7.163(4.0);6.82 6(1.0);6.823(1.0);6.820(1.1);6.818(1.0);5.513(3.1);4.786(0.8);1.538(16.0);0.008(0.6);0.000(19.9);-0.008(0.6) |
| Beispiel I-12: ¹H-NMR(400.0 MHz, CDCl3): |
| δ= 8.497(4.2);8.495(7.6);8.493(8.2);8.491(7.8);8.489(4.0);7.896(3.2);7.892(3.0);7.875(10.3);7.871(10.4) ;7.857(10.2);7.856(9.9);7.836(3.1);7.834(2.8);7.597(1.6);7.581(1.8);7.576(3.4);7.560(3.4);7.555(1.9);7. 538(1.8);7.517(0.6);7.509(12.5);7.495(14.7);7.352(7.5);7.350(7.3);7.338(6.3);7.336(6.2);7.258(76.0);7.1 72(27.0);6.936(1.4);6.933(1.3);6.930(1.6);6.927(1.6);6.915(2.1);6.913(2.2);6.908(2.6);6.906(2.8);6.894( 1.3);6.892(1.3);6.887(3.6);6.881(1.8);6.864(2.8);6.862(2.8);6.858(2.1);6.856(2.2);6.840(2.4);6.834(1.9); 5.486(16.0);5.296(6.0);4.785(6.6);1.553(8.3);0.008(0.9);0.000(32.3);-0.008(1.0) |
| Beispiel I-13: ¹H-NMR(400.0 MHz, CDCl3): |
| δ= 8.166(7.9);8.163(8.0);7.852(5.2);7.847(5.0);7.830(5.9);7.826(5.6);7.651(11.0);7.645(10.9);7.548(5.2) ;7.546(7.2);7.544(4.8);7.526(4.5);7.524(6.2);7.522(4.2);7.518(0.6);7.341 (1.1);7.335(1.1);7.327(1.2);7.3 21(2.0);7.319(1.9);7.313(1.4);7.309(1.2);7.303(1.8);7.301(2.0);7.295(1.2);7.287(1.2);7.281(1.2);7.269(0 .6);7.259(86.0);7.255(0.6);7.209(0.6);7.135(29.3);7.028(1.5);7.023(1.5);7.011(1.6);7.006(3.2);6.999(1.9 );6.995(0.7);6.988(1.9);6.982(2.9);6.977(1.3);6.965(1.2);6.960(1.2);6.814(8.6);6.811(8.5);6.808(8.4);6.8 06(8.0);5.485(14.9);5.484(16.0);5.482(14.5);5.297(2.7);4.783(6.8);1.554(9.9);0.008(1.2);0.000(38.3);-0.008(1.0) |
| Beispiel I-14: ¹H-NMR(400.0 MHz, CDCl3): |
| δ= 8.500(2.6);8.498(4.7);8.496(5.0);8.494(4.8);8.492(2.4);7.900(2.0);7.896(1.9);7.879(6.4);7.876(6.5);7 .868(0.7);7.862(6.2);7.860(6.1);7.840(1.9);7.839(1.7);7.516(0.5);7.511(8.2);7.498(9.7);7.467(0.7);7.462 (5.4);7.460(4.5);7.456(1.8);7.445(2.4);7.440(8.4);7.434(1.1);7.382(1.6);7.376(10.9);7.371(2.9);7.360(2. 5);7.355(10.8);7.349(0.8);7.341 (4.1);7.339(3.9);7.265(0.6);7.264(0.7);7.263(0.8);7.257(56.6);7.253(0.6) ;7.178(16.0);5.428(16.0);5.296(4.1);4.786(4.0);1.548(1.0);0.070(2.3);0.008(0.7);0.000(26.3);-0.008(0.8) |
| Beispiel I-15: ¹H-NMR(400.0 MHz, CDCl3): |
| δ= 8.197(0.9);8.118(3.9);7.971(0.6);7.951(0.8);7.885(1.7);7.881(3.1);7.877(1.7);7.865(2.1);7.861(3.8);7 .857(2.0);7.711(3.3);7.708(7.5);7.706(4.1);7.703(8.4);7.682(5.7);7.663(4.0);7.597(0.9);7.576(1.0);7.517 (0.9);7.450(4.7);7.445(1.9);7.434(2.4);7.428(8.2);7.407(1.2);7.379(1.6);7.374(10.5);7.369(3.0);7.358(2. 3);7.352(5.7);7.309(1.4);7.258(151.8);7.210(1.0);7.148(0.8);7.144(0.9);7.128(0.8);7.124(0.7);7.079(1.5) ;7.075(0.6);7.063(0.6);7.058(1.9);6.994(0.8);6.876(1.6);6.855(1.4);6.832(1.0);6.830(1.1);6.827(1.1);6.8 23(1.2);6.820(1.2);6.818(1.3);6.813(4.6);6.811(4.5);6.808(4.5);6.805(4.4);5.407(16.0);5.297(0.9);5.004( 1.1);4.879(3.7);4.385(0.9);1.534(10.0);1.284(0.7);1.255(0.7);0.008(1.9);0.000(64.5);-0.008(2.3) |
| Beispiel I-16: ¹H-NMR(400.0 MHz, CDCl3): |
| δ= 8.396(6.0);8.394(5.7);8.392(5.4);7.938(2.6);7.917(9.1);7.903(6.1);7.899(5.8);7.881(1.7);7.877(1.7);7 .669(2.7);7.653(2.6);7.647(13.6);7.636(11.5);7.615(2.4);7.482(6.3);7.468(8.4);7.395(6.3);7.394(6.5);7.3 81(4.7);7.380(4.7);7.257(58.5);7.193(20.3);6.875(1.0);5.516(16.0);5.444(0.8);5.294(2.7);4.802(5.7);1.54 8(9.3); 1.257(0.5);0.071 (0.7);0.008(0.8);0.000(23.9);-0.008(0.7) |
| Beispiel I-17: ¹H-NMR(400.0 MHz, CDCl3): |
| δ= 8.086(3.1);7.801(2.1);7.797(2.0);7.780(2.6);7.777(2.5);7.670(4.8);7.664(4.8);7.624(3.2);7.604(2.6);7 .258(14.5);7.242(0.7);7.227(0.8);7.223(0.9);7.220(0.9);7.208(1.0);7.206(1.0);7.201(0.8);7.187(0.8);7.13 2(7.8);6.943(1.1);6.938(1.1);6.921(1.0);6.917(1.7);6.912(1.1);6.895(1.0);6.890(0.9);6.780(3.0);6.777(3. 0);6.774(3.0);6.772(2.8);5.470(5.7);5.468(5.9);5.467(5.5);5.294(14.4);4.792(2.9);4.014(8.8);4.012(16.0) ;4.009(8.5);0.000(6.2) |
| Beispiel I-18: ¹H-NMR(400.0 MHz, CDCl3): |
| δ= 8.385(7.0);8.383(6.8);7.925(3.2);7.903(10.8);7.888(7.0);7.884(6.8);7.867(2.0);7.863(2.2);7.592(1.6); 7.576(1.8);7.571(3.4);7.555(3.4);7.550(1.9);7.534(1.8);7.474(7.7);7.460(10.3);7.388(7.6);7.387(7.7);7.3 75(5.6);7.374(5.5);7.257(75.6);7.169(24.4);6.929(1.3);6.926(1.3);6.923(1.6);6.920(1.6);6.908(2.2);6.906 (2.2);6.901(2.8);6.899(2.9);6.887(1.5);6.884(3.3);6.878(2.9);6.861(3.0);6.859(3.0);6.855(2.1);6.852(2.3) ;6.848(1.1);6.836(2.5);6.830(2.0);5.484(16.0);5.415(0.6);5.295(8.2);4.781(6.5);1.550(16.9);0.008(0.9);0 .000(28.4);-0.008(0.8) |
| Beispiel I-19: ¹H-NMR(400.0 MHz, CDCl3): |
| δ= 8.386(4.4);8.383(4.2);8.382(4.0);7.930(1.3);7.928(2.3);7.926(1.3);7.908(4.1);7.907(7.2);7.905(4.1);7.890(4.4);7.886(4.2);7.869(1.3);7.865(1.4);7.476(4.8);7.462(7.1);7.457(5.6);7.456(4.7);7.452(2.0);7.441 (2.5);7.435(8.7);7.430(1.4);7.391(4.8);7.390(5.0);7.377(4.8);7.371(11.2);7.366(3.1);7.355(2.4);7.350(6. 6);7.344(0.8);7.263(0.5);7.262(0.6);7.262(0.8);7.257(52.0);7.173(15.7);5.426(16.0);5.294(7.4);4.785(4.0 );1.556(2.1);0.008(0.6);0.000(19.6);-0.008(0.6) |
| Beispiel I-20: ¹H-NMR(400.0 MHz, CDCl3): |
| δ= 8.165(6.8);8.164(7.1);8.160(7.1);8.159(6.6);7.847(4.6);7.842(4.4);7.825(5.2);7.820(5.0);7.654(0.7);7 .647(0.8);7.642(10.0);7.637(9.9);7.536(4.8);7.534(6.2);7.532(4.7);7.518(1.0);7.514(4.1);7.512(5.5);7.51 0(4.0);7.269(0.6);7.269(0.6);7.268(0.7);7.267(0.8);7.266(0.9);7.265(1.1);7.265(1.4);7.264(1.6);7.263(2. 1);7.262(2.8);7.259(115.9);7.256(1.7);7.255(1.1);7.254(0.8);7.253(0.6);7.252(0.5);7.209(0.8);7.156(0.5) ;7.114(30.2);6.995(0.6);6.807(7.8);6.805(7.9);6.802(7.8);6.799(7.5);6.750(0.6);6.742(1.0);6.734(5.3);6. 728(0.8);6.716(6.7);6.712(6.8);6.700(0.9);6.694(5.2);6.691(2.3);6.686(1.0);5.489(16.0);5.297(4.9);4.755 (6.0);4.130(0.6);4.112(0.6);3.636(0.5);3.476(0.8);2.043(2.8);1.551(9.0);1.282(0.5);1.276(1.0);1.258(2.7) ;1.240(1.0);0.938(0.6);0.920(1.4);0.902(0.6);0.008(1.4);0.000(53.4);-0.008(1.4) |
| Beispiel I-22: ¹H-NMR(400.0 MHz, CDCl3): |
| δ= 8.373(1.6);8.371(1.6);7.913(0.7);7.891(2.2);7.874(1.6);7.870(1.5);7.849(0.5);7.467(1.7);7.454(2.3);7 .378(1.7);7.364(1.3);7.257(7.6);7.214(0.6);7.199(0.6);7.195(0.5);7.153(4.0);6.936(0.6);6.931(0.6);6.914 (0.6);6.910(1.0);6.905(0.6);6.888(0.5);6.883(0.5);5.471(3.4);5.293(16.0);4.794(1.8);4.009(4.9);4.007(8. 5);4.004(5.0);1.256(0.5);0.000(3.2) |
| Beispiel I-23: ¹H-NMR(400.0 MHz, CDCl3): |
| δ= 8.105(1.8);8.103(2.5);8.101(3.3);8.100(2.4);8.097(1.7);7.819(2.6);7.815(2.4);7.799(3.2);7.795(3.1);7 .684(5.1);7.678(5.0);7.645(3.1);7.644(3.1);7.625(2.5);7.624(2.4);7.338(0.7);7.337(0.7);7.320(0.7);7.318 (0.7);7.269(0.5);7.267(0.7);7.266(0.8);7.259(74.4);7.255(0.5);7.209(0.5);7.168(11.6);7.041(0.5);7.036(0 .5);7.024(0.6);7.018(1.1);7.012(0.7);7.000(0.7);6.995(1.5);6.794(3.2);6.791(3.4);6.788(3.2);6.786(3.2);5 .491(5.2);5.489(5.7);5.488(5.0);5.298(0.6);4.788(2.4);1.538(16.0);0.008(1.0);0.000(32.3);-0.008(0.9) |
| Beispiel I-24: ¹H-NMR(400.0 MHz, CDCl3): |
| δ= 8.164(7.2);8.160(7.3);7.850(4.5);7.846(4.3);7.829(5.1);7.824(4.9);7.646(9.8);7.640(9.8);7.586(1.7);7 .570(1.8);7.565(3.5);7.549(3.5);7.544(2.3);7.541(4.7);7.539(6.5);7.528(1.9);7.517(5.8);7.258(75.8);7.12 0(21.7);6.927(1.3);6.924(1.3);6.921(1.6);6.918(1.7);6.906(2.2);6.904(2.2);6.897(3.0);6.885(1.4);6.882(3 .3);6.876(2.8);6.859(2.9);6.857(2.9);6.853(2.1);6.850(2.3);6.834(2.5);6.828(2.0);6.808(6.9);6.805(7.0);6 .802(7.0);6.800(6.7);5.477(16.0);5.295(2.5);4.768(6.3);1.555(18.5);0.008(0.8);0.000(27.3);-0.008(0.8) |
| Beispiel I-25: ¹H-NMR(400.0 MHz, CDCl3): |
| δ= 8.487(4.4);8.485(8.0);8.484(8.6);8.482(8.5);8.480(4.4);7.892(2.6);7.888(2.3);7.871(10.9);7.868(11.3) ;7.859(10.9);7.858(10.9);7.838(2.4);7.837(2.2);7.517(0.9);7.513(13.7);7.506(0.5);7.499(15.9);7.353(7.8) ;7.351(7.8);7.339(6.7);7.337(6.6);7.267(0.6);7.266(0.6);7.266(0.8);7.265(0.9);7.264(1.1);7.263(1.3);7.2 58(91.8);7.255(2.8);7.254(2.0);7.253(1.6);7.252(1.2);7.252(1.0);7.251(0.8);7.250(0.7);7.249(0.6);7.248( 0.5);7.248(0.5);7.208(0.7);7.184(26.0);5.542(9.0);5.538(16.0);5.534(9.1);5.296(8.1);4.799(6.8);2.044(1. 6);1.550(12.3);1.276(0.6);1.258(1.5);1.240(0.6);0.008(1.3);0.006(0.5);0.005(0.6);0.000(40.9);-0.003(2.0);-0.004(0.8);-0.005(0.6);-0.008(1.2) |
| Beispiel I-26: ¹H-NMR(400.0 MHz, CDCl3): |
| δ= 8.110(1.7);8.108(2.4);8.106(3.3);8.105(2.4);8.102(1.6);7.821(2.6);7.818(2.5);7.801(3.3);7.797(3.1);7 .681(5.1);7.676(5.1);7.642(3.1);7.641(3.1);7.622(2.5);7.620(2.5);7.518(0.8);7.512(2.2);7.507(0.8);7.499 (2.3);7.496(1.2);7.490(2.5);7.482(0.9);7.477(2.4);7.269(0.8);7.268(0.9);7.266(1.2);7.259(114.2);7.255(1 .0);7.254(0.7);7.209(0.6);7.158(11.5);7.101(2.9);7.096(0.9);7.085(1.0);7.079(5.4);7.074(0.9);7.063(0.9); 7.057(2.6);6.995(0.6);6.792(3.4);6.789(3.3);6.786(3.4);6.784(3.2);5.426(8.6);4.769(2.3);1.536(16.0);0.0 08(1.4);0.000(51.6);-0.008(1.5) |
| Beispiel I-27: ¹H-NMR(400.0 MHz, CDCl3): |
| δ= 8.107(2.3);8.105(3.3);8.103(4.2);8.102(3.2);8.099(2.0);7.836(3.1);7.832(2.9);7.816(3.8);7.812(3.6);7 .685(6.2);7.679(6.1);7.651(4.0);7.650(3.8);7.631(3.2);7.630(3.1);7.510(0.8);7.494(0.8);7.489(0.9);7.485 (0.9);7.472(0.9);7.468(0.9);7.463(0.8);7.446(0.8);7.259(65.7);7.176(13.5);7.008(1.0);6.992(1.1);6.985(1 .3);6.969(1.3);6.961 (1.0);6.944(1.0);6.794(3.9);6.792(4.0);6.789(3.9);6.786(3.7);5.458(7.8);5.298(1.1);4 .798(3.2);1.540(16.0);0.008(0.9);0.000(28.3);-0.008(0.8) |
| Beispiel I-28: ¹H-NMR(400.0 MHz, CDCl3): |
| δ= 8.102(1.6);8.100(2.4);8.098(3.0);8.097(2.4);8.095(1.5);7.813(2.3);7.809(2.2);7.793(2.7);7.789(2.7);7 .676(4.8);7.670(4.8);7.634(3.0);7.633(2.9);7.613(2.4);7.612(2.3);7.264(0.5);7.259(31.1);7.246(0.7);7.23 2(0.8);7.228(0.8);7.226(0.8);7.225(0.8);7.213(0.8);7.210(0.9);7.206(0.8);7.192(0.8);7.150(9.5);6.947(1.1);6.942(1.1);6.925(1.0);6.920(1.6);6.916(1.1);6.899(0.9);6.894(0.9);6.786(3.0);6.784(3.1);6.781(3.0);6. 778(3.0);5.473(5.1);5.471(5.2);5.469(5.0);5.297(0.7);4.783(2.5);4.018(8.7);4.015(16.0);4.012(8.6);1.552 (3.0);0.000(13.6) |
| Beispiel I-29: ¹H-NMR(400.0 MHz, CDCl3): |
| δ= 8.397(4.9);7.971(2.2);7.950(8.2);7.937(2.8);7.933(5.0);7.930(2.7);7.912(1.5);7.908(0.9);7.518(2.4);7 .498(4.2);7.494(6.8);7.485(4.6);7.480(9.7);7.476(4.9);7.468(1.7);7.463(4.3);7.414(5.8);7.402(3.9);7.259 (419.7);7.250(0.9);7.102(0.7);7.095(5.5);7.090(1.7);7.078(1.7);7.073(10.0);7.068(1.8);7.057(1.6);7.051( 4.8);6.995(2.2);5.412(16.0);4.876(4.1);1.532(135.8);1.264(0.8);0.882(1.6);0.865(0.6);0.146(0.6);0.008(5 .1);0.000(171.1);-0.008(4.6) |
| Beispiel I-30: ¹H-NMR(400.0 MHz, CDCl3): |
| δ= 8.102(3.9);8.101(4.9);8.099(3.8);7.818(3.6);7.814(3.4);7.798(4.4);7.794(4.3);7.680(7.9);7.675(7.8);7 .640(5.0);7.639(4.8);7.620(3.9);7.466(0.7);7.461(5.2);7.460(4.4);7.455(1.9);7.444(2.5);7.439(8.3);7.433 (1.2);7.381(1.7);7.375(10.8);7.370(2.8);7.359(2.3);7.354(6.0);7.348(0.6);7.258(59.4);7.153(13.9);6.790( 4.9);6.787(5.0);6.784(4.9);6.782(4.6);5.423(16.0);5.296(11.6);4.779(3.9);1.544(8.7);0.008(1.0);0.000(28 .0);-0.008(0.7) |
| Beispiel I-31: ¹H-NMR(400.0 MHz, CDCl3): |
| δ= 8.179(4.6);8.176(4.5);7.866(2.8);7.861(2.7);7.844(3.1);7.840(3.0);7.654(5.6);7.648(5.6);7.552(4.0);7 .530(3.4);7.518(2.0);7.458(5.4);7.442(2.3);7.437(8.2);7.379(1.6);7.373(9.9);7.368(2.7);7.357(2.3);7.352 (5.8);7.292(0.5);7.259(339.7);7.251(1.2);7.247(0.5);7.209(0.7);7.144(14.7);7.037(0.6);6.995(1.9);6.820( 4.1);6.818(4.5);6.815(4.5);6.812(4.3);5.422(16.0);4.765(4.0);1.532(55.4);1.370(0.8);1.333(1.7);1.284(2. 5);1.256(1.3);0.008(4.3);0.000(132.2);-0.008(3.8) |
| Beispiel I-32: ¹H-NMR(400.0 MHz, CDCl3): |
| δ= 8.487(6.9);8.485(7.4);8.483(7.0);8.481(3.7);7.887(2.9);7.884(2.7);7.866(9.1);7.862(9.2);7.848(9.4);7 .847(9.4);7.827(2.9);7.826(2.7);7.517(0.5);7.503(13.0);7.489(15.1);7.346(7.2);7.344(7.1);7.332(6.1);7.3 30(6.0);7.258(84.1);7.254(0.6);7.208(0.6);7.159(21.9);6.755(0.6);6.746(1.1);6.738(5.3);6.733(0.8);6.720 (6.6);6.717(6.6);6.704(0.8);6.698(5.1);6.691(1.0);5.498(16.0);5.296(10.0);4.774(6.2);1.555(10.4);0.008( 1.0);0.000(37.4);-0.008(1.0) |
| Beispiel I-33: ¹H-NMR(400.0 MHz, CDCl3): |
| δ= 7.942(3.2);7.925(3.3);7.921(3.7);7.905(3.5);7.715(7.7);7.710(7.8);7.528(3.9);7.518(5.4);7.444(6.2);7 .423(5.8);7.365(1.9);7.357(2.2);7.334(4.8);7.325(7.4);7.322(7.2);7.320(4.8);7.314(3.0);7.310(4.8);7.292 (1.0);7.290(1.8);7.282(1.3);7.259(985.9);7.250(2.4);7.237(0.8);7.224(1.5);7.209(0.8);6.995(5.4);6.837(4 .0);6.832(4.0);6.829(4.3);6.824(3.9);5.428(16.0);4.871(1.9);1.534(20.7);0.331(0.8);0.157(1.2);0.146(1.0 );0.008(11.2);0.000(368.4);-0.008(9.8);-0.035(0.8);-0.150(1.0) |
| Beispiel I-34: ¹H-NMR(400.0 MHz, CDCl3): |
| δ= 8.173(2.7);8.169(4.8);8.166(2.8);7.899(1.7);7.895(3.2);7.891(1.7);7.878(1.9);7.873(3.5);7.869(1.8);7 .664(7.1);7.658(7.0);7.586(3.3);7.584(4.8);7.582(3.5);7.564(3.0);7.562(4.2);7.560(3.0);7.492(3.9);7.486 (1.6);7.478(4.1);7.477(3.3);7.475(2.3);7.473(2.2);7.470(4.5);7.462(1.7);7.456(4.3);7.449(0.5);7.258(60. 4);7.098(0.6);7.091(5.3);7.086(1.6);7.074(1.8);7.069(9.8);7.064(1.8);7.053(1.7);7.047(4.7);6.833(5.5);6. 831(5.6);6.828(5.6);6.825(5.4);5.404(16.0);5.295(4.7);4.864(4.0);1.547(10.3);0.008(0.7);0.000(24.2);-0.008(0.7) |
| Beispiel I-35: ¹H-NMR(400.0 MHz, CDCl3): |
| δ= 7.694(0.6);7.689(0.6);7.258(12.0);7.177(1.5);7.156(1.5);6.475(2.1);6.470(2.2);6.462(1.6);6.456(0.7); 6.442(1.2);6.436(0.9);5.432(1.4);4.620(1.8);4.605(1.8);3.850(3.4);3.844(14.9);3.813(3.7);3.807(16.0);3. 780(0.7);3.663(0.6);2.118(0.6);1.559(0.7);0.000(5.1) |
| Beispiel I-36: ¹H-NMR(400.0 MHz, CDCl3): |
| δ= 8.174(5.7);8.170(5.7);7.862(3.6);7.858(3.4);7.841(4.1);7.836(4.0);7.666(2.9);7.652(9.4);7.646(16.0); 7.631(10.2);7.610(2.4);7.552(3.6);7.551(5.0);7.549(3.4);7.531(3.2);7.529(4.3);7.258(42.9);7.142(17.1);6 .813(5.5);6.810(5.6);6.807(5.5);6.805(5.1);5.509(15.1);5.295(2.7);4.791(5.3);2.043(0.9);1.558(4.7);1.25 7(0.8);0.008(0.6);0.000(19.4);-0.008(0.6) |
| Beispiel I-37: ¹H-NMR(400.0 MHz, CDCl3): |
| δ= 8.199(8.3);8.195(8.2);7.886(5.4);7.881(5.2);7.864(6.1);7.859(5.8);7.836(3.8);7.816(4.4);7.752(0.6);7 .712(4.0);7.692(4.6);7.660(0.7);7.651(11.2);7.646(11.2);7.605(2.1);7.586(4.2);7.567(2.4);7.553(5.1);7.5 51(7.3);7.550(5.1);7.532(4.4);7.530(6.4);7.528(4.5);7.517(0.6);7.460(2.5);7.441(4.0);7.421(1.6);7.258(9 1.3);7.208(0.8);7.149(28.2);6.817(8.3);6.814(8.5);6.811(8.3);6.809(8.0);5.672(16.0);5.296(4.4);4.786(7. 4);1.920(0.5);1.902(0.5);1.554(3.6);1.282(0.5);1.256(1.3);0.008(1.2);0.000(38.2);-0.008(1.1) |
| Beispiel I-38: ¹H-NMR(400.0 MHz, CDCl3): |
| δ= 8.198(4.5);8.194(4.6);7.874(2.7);7.869(2.6);7.852(3.1);7.848(3.0);7.648(5.7);7.642(5.7);7.549(7.5);7 .528(3.4);7.382(0.8);7.378(0.9);7.369(2.0);7.367(1.9);7.359(1.9);7.355(2.0);7.349(0.6);7.345(0.7);7.341 (0.6);7.334(0.6);7.330(0.7);7.324(7.4);7.322(6.7);7.320(4.7);7.314(2.7);7.310(4.4);7.309(4.3);7.258(49. 6);7.143(13.3);6.819(4.0);6.817(4.3);6.814(4.3);6.811(4.1);5.432(16.0);5.296(1.4);4.779(4.1);1.547(2.6) ;1.371(0.5);1.333(0.5);1.285(0.7);1.282(0.8);1.256(0.6);0.008(0.6);0.000(19.1);-0.008(0.6) |
| Beispiel I-39: ¹H-NMR(400.0 MHz, CDCl3): |
| δ= 9.045(0.6);9.039(16.0);8.625(5.5);8.621(5.5);8.179(4.5);8.178(4.6);8.157(6.0);8.156(6.0);8.023(5.0); 8.018(4.9);8.001(3.8);7.997(3.8);7.588(1.2);7.572(1.3);7.568(2.5);7.551(2.4);7.546(1.5);7.530(1.2);7.51 8(1.0);7.259(167.7);7.199(16.9);6.995(1.0);6.938(1.0);6.935(1.0);6.931(1.1);6.929(1.2);6.915(1.6);6.908 (2.1);6.896(1.0);6.892(2.2);6.887(1.9);6.870(2.0);6.868(2.0);6.861(1.6);6.845(1.7);6.839(1.4);5.489(11. 4);5.297(1.5);4.833(4.1);4.027(0.7);1.557(8.4);1.333(2.2);1.283(3.0);1.256(2.6);0.880(0.5);0.008(2.0);0. 000(62.9);-0.008(1.8) |
| Beispiel I-40: ¹H-NMR(400.0 MHz, CDCl3): |
| δ= 8.414(4.5);8.411(3.8);7.937(1.1);7.916(7.0);7.910(5.5);7.906(5.0);7.889(0.8);7.885(0.8);7.552(3.7);7 .550(3.6);7.548(3.0);7.476(4.7);7.462(6.9);7.403(5.2);7.389(3.8);7.383(1.0);7.379(0.8);7.374(2.0);7.372 (1.9);7.364(2.0);7.360(1.9);7.354(0.6);7.350(0.6);7.344(0.6);7.336(0.6);7.332(0.6);7.326(7.6);7.324(6.5) ;7.322(4.6);7.317(3.1);7.313(4.5);7.312(4.8);7.257(59.6);7.191(12.6);5.439(16.0);5.295(3.3);4.794(4.0); 1.546(4.9);0.008(0.7);0.000(22.5);-0.008(0.6) |
| Beispiel I-41: ¹H-NMR(400.0 MHz, CDCl3): |
| δ= 9.039(15.2);8.644(5.2);8.640(5.2);8.183(4.4);8.182(4.3);8.162(5.8);8.161(5.7);8.034(4.8);8.029(4.7); 8.012(3.6);8.008(3.6);7.518(0.6);7.389(1.2);7.374(1.3);7.368(3.1);7.354(2.4);7.348(2.2);7.334(1.8);7.27 5(5.0);7.268(2.9);7.259(109.3);7.252(2.3);7.245(1.9);7.210(16.1);7.068(0.9);7.065(1.0);7.062(0.9);7.059 (0.9);7.045(1.7);7.040(1.6);7.023(0.8);7.019(0.8);7.017(0.7);6.995(0.6);5.462(16.0);5.297(2.4);4.846(4. 3);4.026(0.5);3.155(0.7);1.559(4.0);1.371(0.6);1.334(1.7);1.286(1.1);1.282(2.5);1.256(2.2);0.008(1.2);0. 000(39.4);-0.008(1.2) |
| Beispiel I-42: ¹H-NMR(400.0 MHz, CDCl3): |
| δ= 8.494(6.8);7.979(2.4);7.975(4.3);7.971(2.4);7.958(4.1);7.954(7.3);7.950(4.2);7.901(8.7);7.881(4.9);7 .583(1.7);7.567(1.9);7.562(3.6);7.547(15.7);7.541(2.5);7.534(15.4);7.525(1.9);7.517(2.9);7.377(7.4);7.3 75(7.5);7.364(6.3);7.362(6.4);7.259(500.2);7.251(1.6);7.208(0.6);6.994(2.7);6.935(1.3);6.932(1.4);6.929 (1.8);6.926(1.6);6.912(2.2);6.905(2.9);6.888(3.9);6.884(2.1);6.863(3.0);6.857(2.3);6.841(2.5);6.835(2.0) ;5.470(16.0);4.881(5.9);1.529(134.3);1.306(0.8);1.265(3.3);0.899(1.8);0.882(6.6);0.864(2.4);0.146(0.6); 0.008(6.1);0.000(203.7);-0.008(5.4);-0.150(0.7) |
| Beispiel I-43: ¹H-NMR(400.0 MHz, CDCl3): |
| δ= 8.109(1.3);8.107(1.9);8.106(2.6);8.104(1.9);8.102(1.3);7.822(2.0);7.818(2.0);7.801(2.5);7.798(2.4);7 .681(4.0);7.676(4.0);7.642(2.4);7.641(2.4);7.622(2.0);7.620(1.9);7.601(0.5);7.585(0.5);7.580(1.0);7.564 (1.0);7.559(0.6);7.543(0.5);7.518(0.5);7.259(91.0);7.209(0.6);7.161(9.0);6.919(0.6);6.910(0.8);6.892(0. 5);6.888(0.9);6.881(0.6);6.865(0.9);6.862(0.8);6.858(0.6);6.856(0.7);6.840(0.7);6.834(0.6);6.791(2.6);6. 789(2.6);6.786(2.5);6.783(2.4);5.483(4.8);5.298(3.2);4.773(1.8);1.536(16.0);0.008(1.1);0.000(38.0);-0.008(1.1) |
| Beispiel I-44: ¹H-NMR(400.0 MHz, CDCl3): |
| δ= 8.540(1.3);8.538(2.3);8.536(2.4);8.534(2.3);8.532(1.2);7.932(1.4);7.928(1.3);7.911(2.8);7.907(2.8);7 .874(2.6);7.872(2.6);7.853(1.3);7.667(0.9);7.661(0.8);7.660(0.9);7.652(0.6);7.650(0.6);7.649(0.8);7.643 (1.0);7.518(0.6);7.514(4.0);7.501(4.7);7.431(1.1);7.426(0.7);7.423(0.6);7.415(1.2);7.413(1.1);7.408(1.5) ;7.361(2.2);7.359(2.2);7.347(1.8);7.345(1.8);7.329(0.5);7.315(1.8);7.310(1.5);7.308(1.7);7.300(4.4);7.2 92(1.5);7.289(1.3);7.284(1.2);7.270(0.6);7.269(0.5);7.268(0.5);7.267(0.6);7.266(0.9);7.265(1.0);7.265(1 .1);7.264(1.3);7.263(1.6);7.259(88.5);7.256(1.4);7.255(0.8);7.254(0.6);7.209(0.7);7.205(8.2);6.995(0.5); 5.589(7.4);5.588(7.4);4.791(1.8);2.044(0.6);1.538(16.0);1.326(0.5);1.258(0.6);0.882(0.8);0.008(1.1);0.0 00(39.3);-0.008(1.0) |
| Beispiel I-45: ¹H-NMR(400.0 MHz, CDCl3): |
| δ= 8.494(3.5);8.492(3.9);7.978(1.5);7.974(2.5);7.969(1.4);7.957(2.6);7.952(4.4);7.948(2.5);7.903(5.3);7 .902(5.3);7.882(3.0);7.880(2.8);7.547(7.5);7.534(8.6);7.457(0.6);7.451(4.9);7.450(4.1);7.446(1.8);7.435 (2.3);7.429(8.2);7.424(1.2);7.380(2.0);7.377(5.6);7.375(13.9);7.369(3.0);7.364(4.0);7.362(4.0);7.358(2. 3);7.353(5.9);7.347(0.6);7.257(61.5);5.412(16.0);5.295(3.4);4.884(3.6);1.536(16.5);0.008(0.8);0.000(26. 6);-0.008(0.7) |
| Beispiel I-46: ¹H-NMR(400.0 MHz, CDCl3): |
| δ= 8.495(7.0);8.493(7.7);8.491(7.4);7.899(2.4);7.896(2.2);7.878(8.9);7.875(9.1);7.864(10.3);7.863(10.3) ;7.843(2.6);7.515(13.5);7.501(16.0);7.357(7.8);7.355(8.2);7.347(1.5);7.343(6.8);7.342(7.0);7.333(1.9);7 .332(1.8);7.325(1.3);7.321(1.2);7.315(1.8);7.313(1.9);7.307(1.2);7.299(1.1);7.293(1.2);7.267(0.7);7.258 (96.6);7.254(0.6);7.209(0.7);7.188(20.4);7.038(1.4);7.032(1.4);7.020(1.4);7.015(3.0);7.009(1.8);6.997(1 .8);6.994(1.4);6.992(2.8);6.986(1.4);6.974(1.2);6.969(1.2);5.495(14.1);5.493(15.4);5.492(13.9);4.802(6. 4);1.555(14.8);0.008(1.2);0.000(43.6);-0.008(1.1) |
| Beispiel I-47: ¹H-NMR(400.0 MHz, CDCl3): |
| δ= 8.176(4.3);7.902(2.5);7.880(2.6);7.669(7.4);7.664(7.3);7.590(4.1);7.569(3.6);7.518(3.4);7.445(5.5);7 .429(2.7);7.423(9.7);7.375(2.0);7.370(13.4);7.364(3.4);7.353(2.9);7.348(7.2);7.292(0.7);7.271(2.1);7.26 8(2.7);7.266(4.2);7.265(4.6);7.259(626.6);7.251 (2.5);7.251 (2.2);7.250(1.8);7.247(1.2);7.244(0.8);7.228( 0.7);6.995(3.4);6.841(5.1);6.838(5.2);6.835(5.1);6.833(5.0);5.405(16.0);4.870(4.0);1.530(136.4);0.146(0 .7);0.008(7.4);0.000(239.8);-0.008(6.4);-0.150(0.7) |
| Beispiel I-48: ¹H-NMR(400.0 MHz, CDCl3): |
| δ= 8.178(4.0);8.175(7.1);8.171(4.1);7.904(2.4);7.899(4.4);7.895(2.5);7.882(2.8);7.878(5.1);7.874(2.7);7 .666(10.3);7.660(10.4);7.588(4.9);7.586(7.0);7.584(5.0);7.572(1.8);7.566(4.4);7.564(6.1);7.562(4.4);7.5 56(1.8);7.551(3.5);7.535(3.5);7.530(2.1);7.518(2.1);7.514(2.0);7.259(349.6);6.995(1.9);6.926(1.4);6.923 (1.4);6.920(1.7);6.917(1.8);6.903(2.3);6.896(3.2);6.883(3.4);6.877(2.9);6.861(3.1);6.858(3.1);6.852(2.4) ;6.836(10.5);6.834(8.8);6.831(9.4);6.828(8.8);5.461(16.0);4.867(5.6);4.130(0.8);4.113(0.8);2.043(3.6);1 .598(2.2);1.276(1.1);1.258(2.3);1.240(1.1);0.008(3.9);0.000(134.4);-0.008(4.0) |
| Beispiel I-49: ¹H-NMR(400.0 MHz, CDCl3): |
| δ= 7.897(2.7);7.881(2.8);7.876(3.1);7.860(2.9);7.710(7.0);7.705(7.1);7.518(0.5);7.446(5.0);7.441(2.0);7 .428(8.2);7.425(9.0);7.419(1.4);7.408(5.6);7.373(1.6);7.367(10.4);7.362(2.8);7.350(2.1);7.346(5.9);7.34 0(0.7);7.300(6.3);7.296(6.5);7.259(90.1);6.995(0.5);6.832(3.5);6.826(3.8);6.824(3.9);6.818(3.4);5.413(1 6.0);5.297(1.2);4.830(3.2);1.561(0.9);1.476(0.6);1.432(2.8);1.428(1.0);1.255(0.6);0.070(2.0);0.008(1.0); 0.000(34.0);-0.008(1.1) |
| Beispiel I-50: ¹H-NMR(400.0 MHz, CDCl3): |
| δ= 8.521(2.5);8.519(4.4);8.517(4.8);8.515(4.5);8.513(2.4);7.916(2.4);7.912(2.3);7.895(5.5);7.891(5.5);7 .865(5.6);7.864(5.6);7.844(2.4);7.843(2.2);7.554(1.6);7.552(2.9);7.550(3.6);7.548(3.6);7.546(2.9);7.544 (1.6);7.510(7.9);7.496(9.4);7.390(0.9);7.386(0.9);7.379(2.3);7.376(1.8);7.373(1.2);7.371(0.8);7.368(2.0) ;7.366(1.4);7.364(2.0);7.357(0.8);7.354(4.8);7.352(4.7);7.349(0.7);7.346(0.9);7.340(3.9);7.338(4.2);7.3 34(0.6);7.329(6.4);7.327(8.0);7.324(4.0);7.318(3.3);7.316(4.2);7.314(6.2);7.262(0.6);7.262(0.7);7.261(0 .9);7.260(1.2);7.257(48.2);7.253(0.5);7.187(15.1);5.440(16.0);5.295(2.2);4.795(3.9);1.546(1.7);0.071(5. 4);0.008(0.7);0.000(22.7);-0.008(0.6) |
| Beispiel I-51: ¹H-NMR(400.0 MHz, CDCl3): |
| δ= 8.137(2.1);7.906(1.0);7.903(1.7);7.899(1.0);7.886(1.2);7.882(2.2);7.878(1.2);7.714(4.1);7.708(4.4);7 .698(0.9);7.690(3.0);7.670(2.3);7.588(2.1);7.568(2.5);7.560(0.6);7.518(1.8);7.453(7.1);7.442(3.3);7.437 (4.2);7.433(9.3);7.405(0.7);7.385(10.6);7.380(11.7);7.352(0.6);7.310(0.7);7.304(1.9);7.298(2.0);7.287(7 .9);7.282(8.3);7.277(3.0);7.273(1.8);7.272(2.0);7.266(9.0);7.266(7.6);7.265(6.7);7.259(319.4);7.253(6.3 );7.252(5.7);7.252(4.9);7.251(4.3);7.250(4.2);7.249(3.9);7.248(3.6);7.248(3.5);7.247(3.4);7.246(3.3);7.2 45(3.2);7.244(3.0);7.242(2.8);7.241(2.8);7.231(7.8);7.209(1.5);6.995(1.8);6.974(0.9);6.970(1.3);6.825(0 .5);6.818(2.9);6.815(2.8);6.812(3.2);6.810(3.0);5.510(8.5);5.136(0.6);4.896(2.2);4.766(15.6);4.750(16.0 );4.723(0.8);2.043(0.5);1.888(6.0);1.873(12.3);1.857(6.1);1.533(79.6);1.505(2.3);1.265(1.3);0.899(0.7); 0.882(2.3);0.864(0.9);0.008(4.2);0.000(140.3);-0.008(5.0);-0.028(3.2);-0.050(0.7) |
| Beispiel I-52: ¹H-NMR(400.0 MHz, CDCl3): |
| δ= 8.172(3.8);8.169(6.6);8.165(3.9);7.897(2.2);7.892(4.3);7.888(2.4);7.875(2.6);7.871(4.7);7.866(2.5);7 .674(0.8);7.669(1.2);7.666(1.2);7.658(9.6);7.652(9.6);7.577(4.7);7.575(6.3);7.573(4.5);7.555(4.0);7.553 (5.8);7.551(4.0);7.517(0.7);7.458(0.5);7.421(0.5);7.258(105.9);6.994(0.6);6.852(0.6);6.850(0.6);6.847(0 .6);6.844(0.6);6.829(7.2);6.827(7.3);6.823(7.4);6.821(7.0);6.750(0.6);6.742(1.1);6.734(5.5);6.729(1.4);6 .726(1.3);6.724(1.0);6.716(7.1);6.712(7.2);6.700(1.0);6.694(5.2);6.686(1.4);6.678(0.6);6.416(0.8);5.474 (16.0);5.437(0.9);5.296(15.7);5.045(0.9);5.011(0.6);4.853(5.4);1.539(19.3);0.008(1.4);0.000(45.3);-0.008(1.2) |
| Beispiel I-53: ¹H-NMR(400.0 MHz, CDCl3): |
| δ= 8.488(4.8);7.973(1.7);7.969(2.9);7.965(1.6);7.952(3.0);7.948(5.1);7.944(2.9);7.898(6.2);7.877(3.5);7 .544(8.9);7.530(10.3);7.500(4.0);7.494(1.6);7.486(4.2);7.478(4.4);7.470(1.7);7.464(4.3);7.374(5.2);7.372(5.1);7.360(4.5);7.358(4.4);7.257(56.1);7.105(0.6);7.098(5.4);7.092(1.6);7.081(1.8);7.076(10.0);7.070( 1.8);7.059(1.6);7.054(4.8);5.413(16.0);5.294(5.2);4.877(4.2);1.541(3.3);0.008(0.7);0.000(24.3);-0.008(0.7) |
| Beispiel I-54: ¹H-NMR(400.0 MHz, CDCl3): |
| δ= 8.092(2.0);8.090(2.9);8.089(3.7);8.087(2.8);8.085(1.9);7.813(2.8);7.810(2.6);7.793(3.5);7.789(3.4);7 .681(5.5);7.676(5.5);7.641(3.6);7.640(3.5);7.620(2.9);7.259(72.1);7.164(12.2);6.790(3.5);6.787(3.5);6.7 84(3.4);6.782(3.3);5.537(4.1);5.533(7.1);5.530(3.9);4.787(3.0);1.537(16.0);0.008(1.0);0.000(31.9);-0.008(0.9) |
| Beispiel I-55: ¹H-NMR(400.0 MHz, CDCl3): |
| δ= 8.484(5.8);8.483(5.2);7.970(2.2);7.965(3.6);7.961(2.1);7.949(3.6);7.944(6.3);7.940(3.6);7.890(7.3);7 .889(7.4);7.869(4.3);7.868(4.2);7.539(11.4);7.525(13.2);7.517(0.6);7.368(6.4);7.366(6.4);7.354(5.5);7.3 52(5.5);7.258(109.2);6.994(0.6);6.747(1.0);6.740(4.9);6.734(0.8);6.721(6.2);6.718(6.2);6.705(0.8);6.700 (4.8);6.692(1.0);5.482(14.4);5.296(16.0);4.867(5.1);1.537(32.2);0.008(1.3);0.000(43.5);-0.008(1.2) |
| Beispiel I-56: ¹H-NMR(400.0 MHz, CDCl3): |
| δ= 8.400(6.7);7.971(3.0);7.949(11.7);7.948(6.9);7.939(4.1);7.935(7.2);7.931(3.7);7.914(2.0);7.578(1.7); 7.561(1.8);7.557(3.5);7.541(3.4);7.535(1.9);7.518(4.1);7.493(8.0);7.479(11.3);7.414(8.1);7.400(5.8);7.3 82(0.6);7.342(0.5);7.259(581.0);7.228(0.6);7.210(0.6);6.995(3.3);6.925(1.4);6.922(1.7);6.919(1.6);6.905 (2.2);6.899(3.0);6.885(3.6);6.878(3.0);6.860(3.0);6.854(2.2);6.838(2.6);6.832(2.1);5.468(16.0);4.878(5. 8);1.532(172.4);0.146(0.6);0.008(7.2);0.000(237.4);-0.008(6.6);-0.150(0.7) |
| Beispiel I-57: ¹H-NMR(400.0 MHz, CDCl3): |
| δ= 8.147(2.3);8.143(2.3);7.837(1.2);7.832(1.2);7.815(1.4);7.811(1.4);7.643(2.6);7.637(2.6);7.525(1.9);7 .504(1.6);7.355(2.4);7.334(3.0);7.259(31.3);7.176(2.9);7.155(2.3);7.117(5.5);6.799(2.0);6.793(2.0);5.62 7(9.2);5.297(4.9);4.748(2.5);3.995(16.0);1.546(4.3);0.000(9.9) |
| Beispiel I-58: ¹H-NMR(400.0 MHz, CDCl3): |
| δ= 8.027(5.0);8.011(5.0);7.660(3.7);7.635(3.6);7.505(0.5);7.437(6.4);7.423(11.4);7.421(5.1);7.417(2.1); 7.406(2.5);7.401(9.2);7.395(1.5);7.360(2.3);7.357(5.7);7.355(13.9);7.349(3.4);7.344(3.6);7.342(3.6);7.3 38(2.4);7.333(6.0);7.327(0.7);7.266(0.7);7.265(0.7);7.258(73.8);5.393(16.0);5.296(4.3);4.911(3.8);1.539 (22.9);1.257(0.6);0.008(0.9);0.000(30.6);-0.008(0.8) |
| Beispiel I-59: ¹H-NMR(400.0 MHz, CDCl3): |
| δ= 8.397(6.1);8.395(6.4);7.961(2.5);7.940(11.1);7.938(6.6);7.931(4.2);7.928(7.0);7.924(3.7);7.910(0.9); 7.906(1.6);7.903(0.9);7.517(1.4);7.485(7.4);7.471(10.7);7.410(7.5);7.408(7.9);7.396(5.3);7.395(5.4);7.2 58(243.0);6.994(1.3);6.753(0.6);6.745(1.1);6.737(5.4);6.718(6.8);6.715(6.9);6.702(0.9);6.697(5.3);6.689 (1.1);6.681(0.5);5.480(16.0);4.865(5.6);1.534(96.9);0.008(3.0);0.000(97.0);-0.008(2.6) |
| Beispiel I-60: ¹H-NMR(400.0 MHz, CDCl3): |
| δ= 9.044(11.9);8.659(3.7);8.657(4.1);8.654(4.0);8.653(3.9);8.189(3.4);8.188(3.5);8.168(4.5);8.166(4.6); 8.040(3.9);8.035(3.9);8.018(3.0);8.014(3.0);7.560(1.3);7.556(3.0);7.554(2.8);7.552(2.5);7.518(1.1);7.38 8(0.7);7.384(0.7);7.379(0.5);7.377(1.0);7.375(1.6);7.372(1.6);7.365(1.8);7.361(1.8);7.354(0.7);7.353(0. 9);7.351(0.7);7.346(0.6);7.341(0.6);7.336(6.9);7.334(5.9);7.332(4.1);7.326(2.3);7.323(3.7);7.321(3.4);7. 275(0.5);7.274(0.5);7.273(0.6);7.272(0.6);7.271(0.7);7.271(0.8);7.270(0.9);7.269(1.0);7.268(1.2);7.267( 1.3);7.267(1.5);7.266(1.8);7.265(2.1);7.264(2.7);7.263(3.6);7.259(199.4);7.255(3.2);7.254(2.1);7.254(1. 4);7.253(1.0);7.252(0.8);7.251(0.6);7.250(0.6);7.234(0.6);7.230(10.8);7.226(0.6);6.995(1.1);5.447(13.6) ;4.888(2.0);4.148(1.1);4.130(3.4);4.113(3.4);4.095(1.1);2.050(0.6);2.043(16.0);1.598(0.6);1.283(0.7);1. 276(5.2);1.258(10.5);1.241(4.9);0.008(2.2);0.006(0.6);0.000(81.2);-0.005(1.0);-0.006(0.8);-0.007(0.7);-0.008(2.3) |
| Beispiel I-61: ¹H-NMR(400.0 MHz, CDCl3): |
| δ= 8.512(1.5);8.510(2.7);8.508(2.9);8.506(2.7);8.504(1.4);7.920(1.1);7.916(1.0);7.899(3.6);7.895(3.7);7 .881(3.5);7.880(3.5);7.860(1.0);7.859(1.0);7.525(4.8);7.518(0.6);7.511(5.6);7.366(2.7);7.364(2.6);7.353 (2.3);7.351(2.2);7.324(0.5);7.320(0.6);7.305(0.5);7.299(0.6);7.259(83.1);7.255(0.7);7.220(9.6);7.209(0. 5);5.488(4.3);5.487(5.2);5.485(4.3);4.823(2.1);1.543(16.0);0.008(1.1);0.004(0.5);0.002(1.4);0.000(34.9) ;-0.008(1.0) |
| Beispiel I-62: ¹H-NMR(400.0 MHz, CDCl3): |
| δ= 8.226(5.5);8.222(5.7);8.196(3.2);8.193(3.4);8.175(3.4);8.172(3.5);7.961(2.7);7.942(3.2);7.916(3.2);7 .911(3.2);7.894(3.6);7.889(3.6);7.711(1.8);7.692(3.4);7.673(2.4);7.665(6.8);7.660(6.5);7.574(5.0);7.552 (4.0);7.531(1.9);7.518(3.5);7.492(1.4);7.259(521.2);7.223(0.9);7.191(15.3);6.995(2.8);6.836(5.1);6.832( 4.8);6.830(4.8);5.901(16.0);5.523(1.0);5.298(1.9);4.806(5.2);1.530(124.6);1.332(0.8);1.284(1.2);1.256(1.0);0.145(0.7);0.008(6.5);0.000(189.9);-0.008(5.8);-0.150(0.7) |
| Beispiel I-63: ¹H-NMR(400.0 MHz, CDCl3): |
| δ= 8.199(2.4);8.195(4.1);8.192(2.4);7.926(1.5);7.922(2.7);7.918(1.5);7.905(1.6);7.900(3.0);7.896(1.6);7 .671(6.1);7.666(6.1);7.597(3.0);7.595(4.1);7.593(3.1);7.576(2.8);7.573(5.6);7.572(6.6);7.551(4.5);7.517 (0.5);7.440(5.3);7.435(5.6);7.290(3.7);7.284(3.6);7.269(3.6);7.264(4.1);7.258(97.8);7.252(0.6);6.994(0. 5);6.842(4.9);6.840(5.0);6.837(4.9);6.834(4.8);5.507(16.0);4.884(3.6);1.533(21.7);0.008(1.3);0.000(44.2 );-0.008(1.1) |
| Beispiel I-64: ¹H-NMR(400.0 MHz, CDCl3): |
| δ= 9.071(14.0);9.036(1.8);8.585(4.5);8.242(0.7);8.237(0.7);8.231(0.8);8.222(4.3);8.201(5.9);8.200(5.8); 8.132(2.3);8.127(4.4);8.123(2.3);8.110(1.5);8.106(2.8);8.101(1.4);7.833(0.6);7.828(0.6);7.807(0.5);7.51 8(0.9);7.447(4.8);7.442(1.8);7.431(2.3);7.426(8.6);7.420(1.3);7.382(1.7);7.376(11.0);7.371(3.0);7.360(2 .2);7.355(5.8);7.349(0.9);7.345(1.7);7.339(0.8);7.329(1.4);7.323(7.3);7.319(1.9);7.310(5.4);7.304(1.0);7 .294(0.6);7.288(1.5);7.259(160.5);6.995(0.9);5.414(16.0);4.925(3.7);4.676(6.1);3.016(1.3);1.552(2.7);1. 370(0.8);1.286(1.2);1.255(0.8);0.008(1.8);0.000(59.7);-0.008(1.6) |
| Beispiel I-65: ¹H-NMR(400.0 MHz, CDCl3): |
| δ= 8.399(4.1);7.974(2.2);7.954(4.0);7.953(7.3);7.951(4.1);7.940(2.4);7.936(4.2);7.932(2.3);7.919(0.6);7 .914(1.2);7.911 (0.7);7.517(1.5);7.496(4.7);7.482(6.7);7.449(5.1);7.444(1.9);7.432(2.5);7.427(8.7);7.422 (1.5);7.418(5.0);7.417(5.1);7.405(3.5);7.403(3.5);7.377(1.7);7.372(11.2);7.366(3.0);7.355(2.3);7.350(6. 1);7.344(0.7);7.259(274.2);6.995(1.5);5.411(16.0);4.882(3.6);2.043(1.4);1.531(81.3);1.276(0.5);1.258(1. 0);0.008(3.4);0.000(113.4);-0.008(3.0) |
| Beispiel I-66: ¹H-NMR(400.0 MHz, CDCl3): |
| δ= 7.797(8.8);7.780(8.7);7.653(11.8);7.647(11.9);7.543(1.7);7.527(1.9);7.522(3.5);7.518(1.0);7.506(3.5) ;7.501(2.1);7.485(1.9);7.319(5.3);7.318(5.4);7.295(5.2);7.294(5.3);7.293(5.5);7.267(0.9);7.266(1.0);7.2 59(110.0);6.995(0.6);6.910(1.4);6.908(1.4);6.904(1.8);6.901(1.8);6.889(2.1);6.887(2.3);6.881(3.3);6.869 (3.4);6.863(2.6);6.860(2.1);6.848(3.0);6.846(3.0);6.842(2.0);6.839(2.3);6.823(2.7);6.817(2.2);6.799(8.1) ;6.797(8.3);6.794(8.3);6.791(8.0);5.447(16.0);5.296(2.0);4.896(5.9);1.543(22.2);1.257(0.6);0.008(1.4);0 .000(44.2);-0.008(1.2) |
| Beispiel I-67: ¹H-NMR(400.0 MHz, CDCl3): |
| δ= 8.108(1.0);8.106(1.4);8.105(1.8);8.103(1.3);8.101(1.0);7.838(1.5);7.834(1.4);7.817(1.8);7.814(1.8);7 .690(2.8);7.684(2.8);7.658(1.7);7.657(1.7);7.638(1.4);7.636(1.4);7.259(76.5);7.255(0.5);7.192(6.8);6.79 9(1.9);6.797(1.9);6.794(1.9);6.791(1.8);5.482(2.6);5.480(3.2);5.478(2.7);4.810(1.3);1.533(16.0);0.008(1 .1);0.004(0.6);0.002(1.5);0.000(33.4);-0.003(1.4);-0.008(1.0) |
| Beispiel I-68: ¹H-NMR(400.0 MHz, CDCl3): |
| δ= 7.797(5.3);7.779(5.3);7.657(7.1);7.651(7.2);7.518(1.0);7.427(0.7);7.421(4.9);7.420(4.0);7.416(1.8);7 .405(2.4);7.400(9.0);7.394(1.5);7.361(1.9);7.355(11.6);7.350(3.0);7.339(2.2);7.334(5.6);7.324(3.3);7.32 2(3.3);7.299(3.2);7.298(3.4);7.259(179.7);6.995(1.0);6.804(4.9);6.802(5.0);6.798(5.0);6.796(4.9);5.390( 16.0);4.898(3.6);1.535(43.9);1.255(0.6);0.008(2.1);0.000(70.1);-0.008(2.0) |
| Beispiel I-69: ¹H-NMR(400.0 MHz, CDCl3): |
| δ= 9.040(10.6);8.622(3.4);8.621(3.6);8.618(3.6);8.616(3.4);8.179(3.1);8.178(3.1);8.158(4.1);8.156(4.1); 8.022(3.5);8.017(3.4);8.000(2.7);7.996(2.7);7.459(3.9);7.458(3.2);7.454(1.4);7.443(1.8);7.438(6.3);7.43 2(1.0);7.385(1.2);7.379(8.0);7.374(2.2);7.362(1.9);7.357(4.8);7.352(0.8);7.259(78.8);7.254(0.5);7.199(1 2.0);5.431(11.8);5.297(16.0);4.839(2.8);1.427(0.5);1.422(0.8);1.371(1.3);1.333(8.5);1.286(2.7);1.282(12 .0);1.256(6.4);1.232(0.8);0.880(1.4);0.862(0.6);0.008(0.9);0.000(32.7);-0.008(1.0) |
| Beispiel I-70: ¹H-NMR(400.0 MHz, CDCl3): |
| δ= 9.073(13.3);8.606(4.7);8.229(3.7);8.228(3.6);8.207(6.1);8.206(6.1);8.149(2.5);8.145(4.6);8.141(2.4); 8.128(1.5);8.123(2.7);8.119(1.4);7.518(1.7);7.387(1.2);7.372(1.8);7.367(3.1);7.352(2.5);7.346(2.0);7.33 3(1.8);7.292(0.7);7.259(316.7);7.247(3.8);7.232(1.5);7.227(1.6);7.225(1.4);7.066(1.0);7.062(1.0);7.045( 1.8);7.040(1.5);7.024(0.9);7.020(0.8);6.995(1.7);5.446(16.0);4.934(4.0);1.536(65.6);1.333(1.0);1.284(1. 6);1.256(1.8);0.008(3.8);0.000(123.8);-0.008(3.4) |
| Beispiel I-71: ¹H-NMR(400.0 MHz, CDCl3): |
| δ= 7.794(6.1);7.777(6.1);7.654(8.2);7.648(8.3);7.471(4.0);7.466(1.6);7.458(4.3);7.455(2.2);7.449(4.6);7 .442(1.8);7.436(4.4);7.429(0.5);7.320(3.7);7.319(3.8);7.296(3.8);7.294(3.8);7.268(0.5);7.266(0.8);7.258 (86.2);7.086(0.7);7.078(5.5);7.073(1.7);7.069(0.6);7.062(1.8);7.056(10.0);7.051(1.8);7.040(1.6);7.035(4 .8);7.027(0.5);6.799(5.7);6.796(5.8);6.793(5.8);6.791(5.7);5.391(16.0);5.296(1.4);4.894(4.1);1.543(18.2 );1.256(0.6);0.008(1.0);0.000(34.6);-0.008(0.9) |
| Beispiel I-72: ¹H-NMR(400.0 MHz, CDCl3): |
| δ= 9.045(16.0);8.635(5.2);8.634(5.8);8.631(5.6);8.629(5.4);8.190(4.8);8.189(4.9);8.169(6.3);8.168(6.3); 8.037(5.4);8.033(5.3);8.016(4.2);8.011(4.1);7.676(2.8);7.655(9.8);7.638(8.6);7.617(2.5);7.518(1.2);7.25 9(205.7);7.250(0.5);7.220(18.6);6.995(1.2);5.521(13.5);4.850(4.5);4.027(0.8);3.227(1.0);1.548(21.2);1.3 33(3.3);1.283(4.6);1.257(3.6);0.880(0.8);0.008(2.4);0.000(83.2);-0.008(2.4) |
| Beispiel I-73: ¹H-NMR(400.0 MHz, CDCl3): |
| δ= 8.417(5.1);8.414(4.9);7.952(1.7);7.950(3.2);7.948(1.7);7.930(4.6);7.929(8.3);7.927(4.6);7.906(5.0);7 .902(4.6);7.885(1.8);7.881(1.9);7.518(1.0);7.492(5.3);7.478(7.5);7.413(5.5);7.411(5.6);7.399(4.0);7.398 (3.9);7.359(0.7);7.354(0.7);7.341(1.4);7.335(1.3);7.326(1.0);7.319(0.9);7.313(1.2);7.309(0.8);7.300(0.8) ;7.294(0.8);7.273(0.6);7.272(0.6);7.271(0.6);7.270(0.7);7.270(0.8);7.269(0.9);7.268(1.0);7.267(1.2);7.2 66(1.3);7.266(1.5);7.265(1.8);7.264(2.3);7.263(3.2);7.259(172.2);7.254(2.2);7.253(1.6);7.252(1.2);7.252 (1.0);7.251(0.8);7.250(0.6);7.249(0.5);7.222(21.1);7.209(1.1);6.995(1.0);5.489(8.8);5.487(10.8);5.485(9 .4);5.298(0.6);4.819(4.5);1.537(16.0);1.258(0.5);0.008(2.0);0.000(75.0);-0.008(2.2) |
| Beispiel I-74: ¹H-NMR(400.0 MHz, CDCl3): |
| δ= 8.394(2.2);8.377(1.0);8.373(1.0);8.348(2.8);8.345(2.6);7.946(0.8);7.925(3.2);7.906(2.2);7.904(3.7);7 .902(2.1);7.886(0.9);7.882(0.9);7.864(0.6);7.857(2.3);7.854(2.2);7.836(1.2);7.832(1.2);7.518(0.7);7.480 (3.9);7.467(5.5);7.405(1.4);7.404(1.4);7.398(3.0);7.397(3.0);7.392(1.0);7.390(1.0);7.385(2.1);7.383(2.1) ;7.259(119.0);7.237(0.7);7.223(0.8);7.216(0.9);7.209(0.8);7.201(0.9);7.197(0.8);7.182(0.8);7.123(3.0);7 .044(6.5);6.995(0.9);6.961(0.5);6.953(0.9);6.939(2.0);6.934(1.6);6.920(1.1);6.916(2.2);6.912(2.8);6.908 (1.2);6.891(2.7);6.886(2.1);6.869(0.6);6.864(0.7);5.472(5.1);5.429(0.6);5.414(1.2);5.398(0.6);4.588(2.8) ;4.573(2.6);4.029(8.6);4.027(12.4);4.024(8.2);4.012(8.6);4.010(16.0);4.007(8.5);1.545(3.4);1.255(1.0);0 .008(1.4);0.000(51.0);-0.008(1.4) |
| Beispiel I-75: ¹H-NMR(400.0 MHz, CDCl3): |
| δ= 8.029(8.2);8.013(8.2);7.732(0.6);7.730(0.6);7.657(6.0);7.632(6.0);7.545(1.8);7.529(1.9);7.525(3.7);7 .517(1.0);7.509(3.6);7.504(2.9);7.488(2.0);7.435(10.5);7.421(14.8);7.356(7.6);7.354(7.9);7.342(5.5);7.3 40(5.5);7.269(0.7);7.269(0.7);7.268(0.7);7.267(0.7);7.265(1.3);7.264(1.5);7.258(132.2);7.251(0.5);6.994 (0.7);6.911(1.5);6.908(1.5);6.904(1.8);6.902(1.9);6.890(2.2);6.888(2.3);6.883(3.0);6.881(3.5);6.870(3.3) ;6.863(2.5);6.860(2.2);6.848(3.0);6.846(3.2);6.842(2.1);6.840(2.4);6.824(2.5);6.818(2.0);5.451(16.0);5. 296(1.5);4.907(6.3);1.540(36.8);1.258(0.7);0.008(1.4);0.000(53.5);-0.008(1.5) |
| Beispiel I-76: ¹H-NMR(400.0 MHz, CDCl3): |
| δ= 8.520(2.6);8.518(3.5);8.517(3.9);8.001(1.6);7.996(2.8);7.992(1.6);7.979(2.5);7.975(4.5);7.971(2.5);7 .912(5.2);7.910(5.2);7.890(3.2);7.889(3.1);7.588(3.9);7.567(4.5);7.553(7.9);7.540(9.2);7.517(0.7);7.445 (5.4);7.440(5.8);7.383(4.5);7.381(4.5);7.369(3.8);7.368(3.8);7.302(3.6);7.297(3.4);7.281(3.2);7.276(3.1) ;7.258(123.4);6.994(0.7);5.515(16.0);4.900(3.8);1.531(35.1);0.008(1.7);0.000(52.5);-0.008(1.4) |
| Beispiel I-77: ¹H-NMR(400.0 MHz, CDCl3): |
| δ= 8.027(5.4);8.011(5.4);7.658(4.0);7.634(4.0);7.517(0.7);7.505(0.6);7.503(0.6);7.492(0.6);7.481(0.5);7 .474(3.8);7.469(1.7);7.461(4.0);7.458(2.4);7.455(2.2);7.452(4.5);7.444(1.7);7.439(4.6);7.436(7.8);7.422 (9.7);7.355(5.2);7.353(5.4);7.341(3.8);7.340(3.8);7.266(0.8);7.258(87.9);7.086(0.6);7.079(5.3);7.074(1. 7);7.062(1.7);7.057(9.7);7.052(1.8);7.041(1.5);7.035(4.6);5.396(16.0);5.296(2.8);4.904(4.4);1.538(25.2) ;1.258(0.6);0.008(1.2);0.000(36.6);-0.008(1.0) |
| Beispiel I-78: ¹H-NMR(400.0 MHz, CDCl3): |
| δ= 9.051(15.7);9.039(1.8);8.648(4.8);8.647(5.2);8.644(5.1);8.643(4.9);8.586(0.6);8.401(1.0);8.194(4.9); 8.193(4.6);8.173(6.5);8.171(6.0);8.045(5.0);8.040(4.9);8.023(4.0);8.019(4.1);8.003(0.6);7.998(0.6);7.59 0(4.1);7.569(4.7);7.520(3.1);7.453(5.8);7.448(6.2);7.312(0.7);7.307(3.9);7.302(3.5);7.286(3.7);7.281(3. 7);7.270(3.4);7.262(553.5);7.255(2.3);7.254(1.9);7.253(1.4);7.252(0.9);7.252(0.9);7.251(0.9);7.250(0.8) ;7.249(0.7);7.248(0.6);7.248(0.6);7.247(0.6);7.234(0.6);7.225(17.8);7.212(1.1);7.136(1.9);6.998(3.0);5. 537(16.0);5.302(2.0);4.849(4.2);1.551(125.9);1.333(2.6);1.284(3.8);1.255(2.0);0.882(0.7);0.146(0.6);0.0 08(6.5);0.000(207.3);-0.006(1.5);-0.007(1.3);-0.008(5.5);-0.150(0.6) |
| Beispiel I-79: ¹H-NMR(400.0 MHz, CDCl3): |
| δ= 7.798(8.3);7.780(8.2);7.648(11.1);7.642(11.2);7.518(0.7);7.309(5.2);7.308(5.1);7.285(4.9);7.283(5.2) ;7.270(0.6);7.268(0.7);7.259(116.9);6.995(0.6);6.796(7.8);6.794(8.0);6.791(7.9);6.788(7.7);6.739(0.6);6 .731(1.1);6.723(5.5);6.717(1.0);6.705(6.8);6.702(6.9);6.689(0.8);6.683(5.2);6.676(1.1);5.462(16.0);5.29 7(4.8);4.882(5.6);2.042(0.6);1.541(36.3);1.258(0.9);0.008(1.4);0.000(46.7);-0.008(1.3) |
| Beispiel I-80: ¹H-NMR(400.0 MHz, CDCl3): |
| δ= 8.045(4.9);8.028(4.9);7.669(3.8);7.645(3.7);7.535(4.2);7.518(6.2);7.514(5.2);7.445(6.5);7.431(10.1);7.424(6.6);7.378(1.0);7.364(4.8);7.352(3.6);7.309(1.3);7.274(5.9);7.269(7.4);7.259(1013.5);7.249(4.5);6 .995(5.4);5.499(16.0);4.923(4.1);1.530(299.2);0.146(1.3);0.008(12.5);0.000(401.7);-0.008(10.7);-0.150(1.2) |
| Beispiel I-81: ¹H-NMR(400.0 MHz, CDCl3): |
| δ= 7.811(5.5);7.794(5.4);7.661(7.4);7.655(7.5);7.529(4.0);7.518(2.7);7.509(4.7);7.428(6.1);7.423(6.5);7 .330(3.4);7.307(3.4);7.305(3.4);7.274(4.9);7.269(5.9);7.259(480.7);7.248(3.6);6.995(2.5);6.809(5.1);6.8 07(5.2);6.804(5.2);6.801(5.2);5.495(16.0);4.911(3.8);1.532(126.2);1.258(0.6);0.146(0.5);0.008(5.8);0.00 0(185.6);-0.008(4.8);-0.150(0.6) |
| Beispiel I-82: ¹H-NMR(400.0 MHz, CDCl3): |
| δ= 8.423(4.0);8.421(4.2);7.982(0.9);7.961(7.2);7.959(6.9);7.955(5.6);7.952(3.0);7.934(0.7);7.580(4.0);7 .559(4.6);7.517(1.0);7.500(5.2);7.486(7.3);7.443(5.9);7.438(6.2);7.420(5.6);7.407(3.8);7.293(3.7);7.287 (3.6);7.272(3.7);7.266(4.0);7.258(185.5);7.250(0.8);7.249(0.6);6.994(1.0);5.513(16.0);4.898(3.8);1.532( 59.6);0.008(2.3);0.000(74.0);-0.008(2.1) |
| Beispiel I-83: ¹H-NMR(400.0 MHz, CDCl3): |
| δ= 9.071(14.2);8.584(4.8);8.221(4.0);8.220(4.0);8.200(6.4);8.198(6.3);8.131(2.6);8.126(4.8);8.122(2.5); 8.109(1.7);8.105(3.1);8.100(1.6);7.518(1.8);7.498(3.6);7.493(1.5);7.485(3.8);7.476(4.2);7.468(1.6);7.46 3(4.0);7.259(318.6);7.108(0.5);7.100(4.9);7.095(1.5);7.084(1.7);7.079(9.1);7.074(1.6);7.062(1.4);7.057( 4.3);6.995(1.8);5.417(16.0);5.298(1.4);4.917(4.2);1.536(33.0);1.370(0.8);1.333(1.0);1.284(1.7);1.256(1. 7);0.008(3.8);0.000(120.5);-0.008(3.3) |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man
75 Gew.-Teile einer Verbindung der allgemeinen Formel (I),
10 " ligninsulfonsaures Calcium,
5 " Natriumlaurylsulfat,
3 " Polyvinylalkohol und
7 " Kaolin
mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man
25 Gew.-Teile einer Verbindung der allgemeinen Formel (I),
5 " 2,2'-dinaphthylmethan-6,6'-disulfonsaures Natrium,
2 " oleoylmethyltaurinsaures Natrium,
1 " Polyvinylalkohol,
17 " Calciumcarbonat und
50 " Wasser
auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigten zahlreiche erfindungsgemäße Verbindungen bei einer Aufwandmenge von 0,32 kg oder weniger pro Hektar eine mindestens 80%-ige Wirkung gegen eine Vielzahl bedeutender Schadpflanzen.

Gleichzeitig lassen erfindungsgemäße Verbindungen Gramineenkulturen wie Gerste, Weizen, Roggen, Hirse, Mais oder Reis im Vorauflaufverfahren selbst bei hohen Wirkstoffdosierungen praktisch ungeschädigt. Einige Substanzen schonen darüber hinaus auch zweikeimblättrige Kulturen wie Soja, Baumwolle, Raps, Zuckerrüben oder Kartoffeln. Die erfindungsgemäßen Verbindungen zeigen teilweise eine hohe Selektivität und eignen sich deshalb im Vorauflaufverfahren zur Bekämpfung von unerwünschtem Pflanzenwuchs in landwirtschaftlichen Kulturen. Die nachfolgenden Tabellen zeigen beispielhaft die herbizide Wirkung der erfindungsgemäßen Verbindungen im Vorauflauf, wobei die herbizide Wirkung in Prozent angegeben ist.

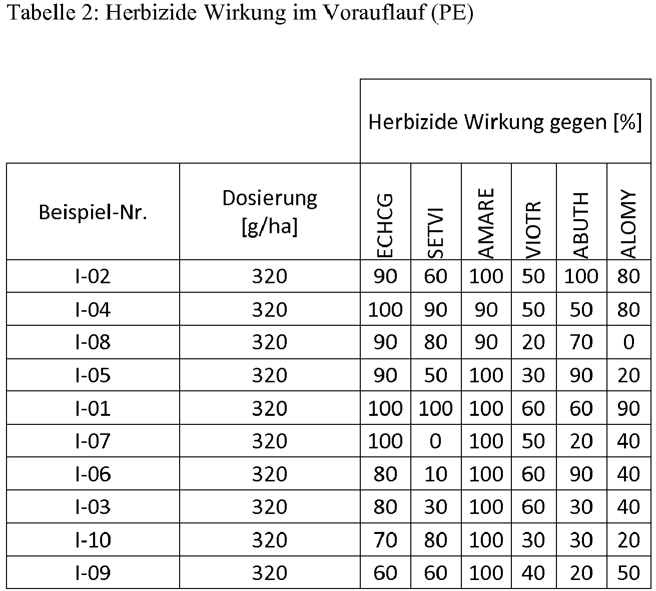

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigten zahlreiche erfindungsgemäße Verbindungen bei einer Aufwandmenge von 0,08 kg oder weniger pro Hektar eine mindestens 80%-ige Wirkung gegen eine Vielzahl bedeutender Schadpflanzen. Gleichzeitig lassen erfindungsgemäße Verbindungen Gramineenkulturen wie Gerste, Weizen, Roggen, Hirse, Mais oder Reis im Nachauflaufverfahren selbst bei hohen Wirkstoffdosierungen praktisch ungeschädigt. Einige Substanzen schonen darüber hinaus auch zweikeimblättrige Kulturen wie Soja, Baumwolle, Raps, Zuckerrüben oder Kartoffeln. Die erfindungsgemäßen Verbindungen zeigen teilweise eine hohe Selektivität und eignen sich deshalb im Nachauflaufverfahren zur Bekämpfung von unerwünschtem Pflanzenwuchs in landwirtschaftlichen Kulturen. Die nachfolgenden Tabellen zeigen beispielhaft die herbizide Wirkung der erfindungsgemäßen Verbindungen im Nachauflauf, wobei die herbizide Wirkung in Prozent angegeben ist.

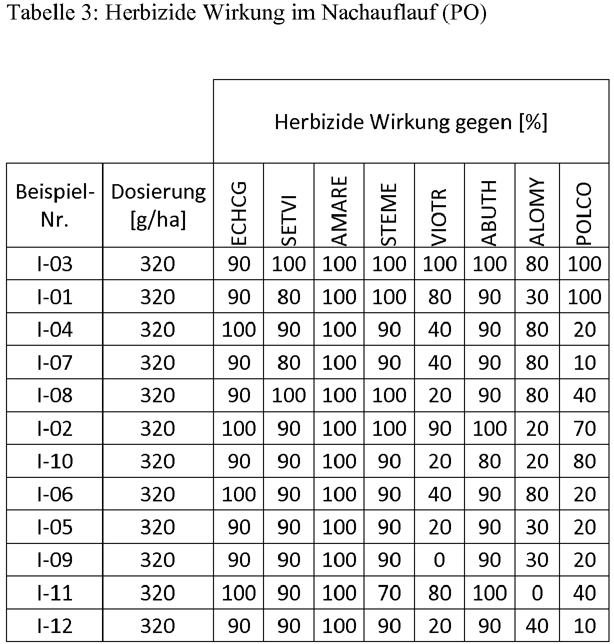

## Patentansprüche

1. Verbindungen der Formel (I), 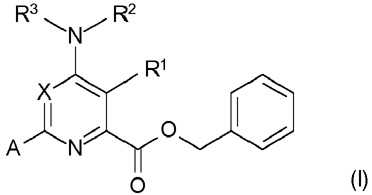 deren N-Oxide oder deren agrochemisch verträglichen Salze, in welchen
A einen Rest aus der Gruppe bestehend aus A1 bis A20 bedeutet, 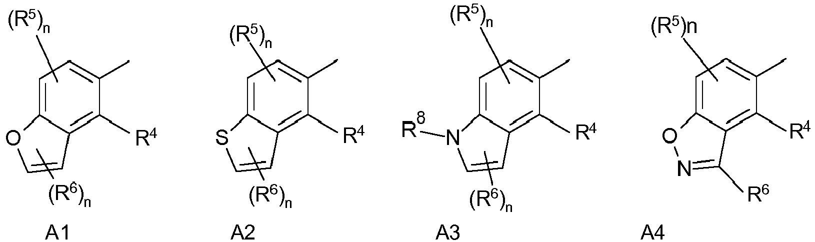 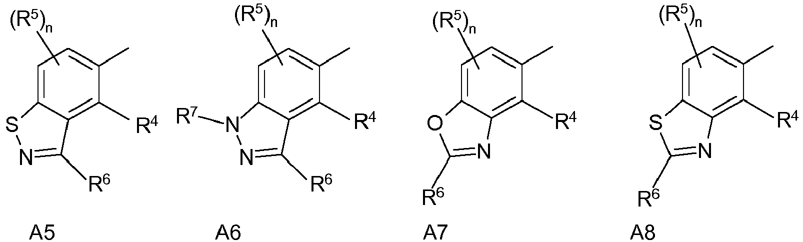 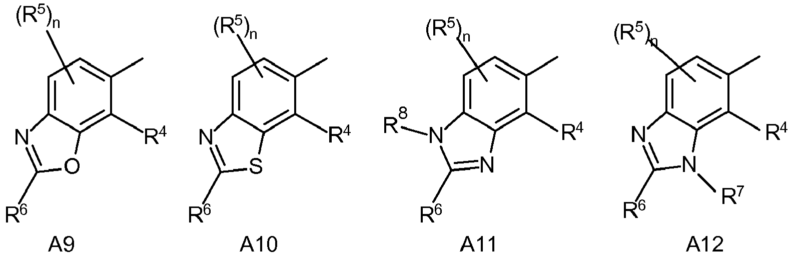 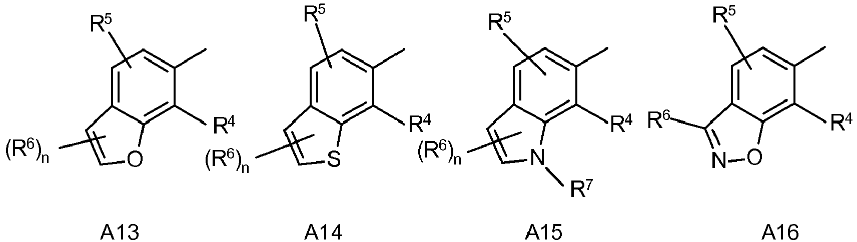
R¹ Chlor, Fluor oder Wasserstoff bedeutet,
R² Wasserstoff bedeutet,
R³ Wasserstoff bedeutet,
R⁴ Wasserstoff, Halogen, OH, NH₂, CN, (C₁-C₃)-Alkyl, (C₁-C₃)-Alkoxy, (C₁-C₃)-Alkylamino oder Cyclopropyl bedeutet,
R⁵ Wasserstoff, Halogen, OH, NH₂, CN, (C₁-C₃)-Alkyl, (C₁-C₃)-Alkoxy, Cyclopropyl oder Vinyl bedeutet,
R⁶ Wasserstoff, Halogen, (C₁-C₃)-Alkyl, (C₁-C₃)-Alkoxy, (C₁-C₃)-Alkylthio, Cyclopropyl, (C₁-C₃)-Alkylamino oder Phenyl bedeutet,
R⁷ Wasserstoff, (C₁-C₆)-Alkyl, (C₁-C₄)-Alkylcarbonyl, (C₁-C₆)-Alkoxycarbonyl oder Phenyl bedeutet,
X N, CH oder CF bedeutet, und
n bedeutet 0, 1 oder 2.

2. Verbindungen der Formel (I) gemäß Anspruch 1, in welchen A einen Rest aus der Gruppe bestehend A1 bis A3, A7 bis A15 und A17 bis A18 bedeutet.

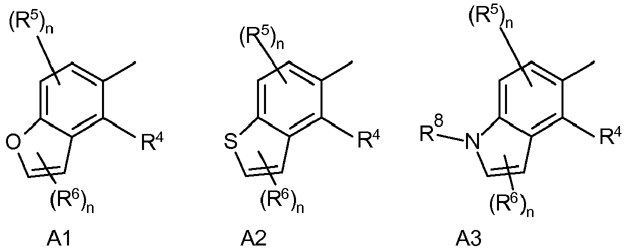

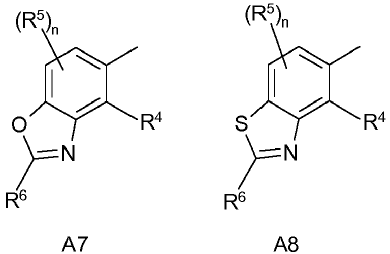

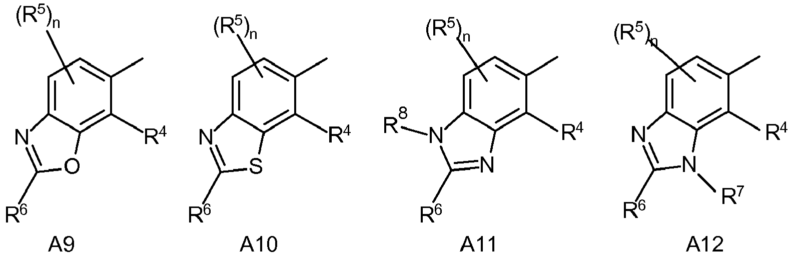

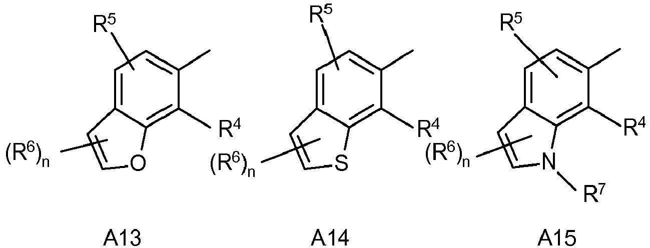

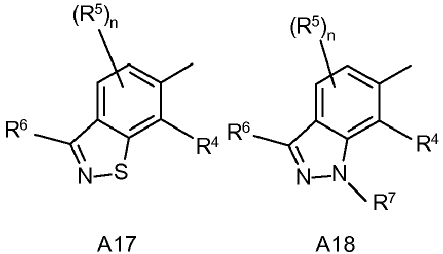

3. Verbindungen der Formel (I) gemäß Anspruch 1 oder 2, in welchen R¹ Chlor bedeutet.

4. Verbindungen der Formel (I) gemäß einem der vorherigen Ansprüche, in welchen R² und R³ Wasserstoff bedeuten.

5. Verbindungen der Formel (I) gemäß einem der vorherigen Ansprüche, in welchen R⁴ Wasserstoff oder Fluor bedeutet.

6. Verbindungen der Formel (I) gemäß einem der vorherigen Ansprüche, in welchen R⁵ Wasserstoff oder Halogen bedeutet.

7. Verbindungen der Formel (I) gemäß einem der vorherigen Ansprüche, in welchen R⁶ Wasserstoff, Halogen oder (C₁-C₃)-Alkyl bedeutet.

8. Verbindungen der Formel (I) gemäß einem der vorherigen Ansprüche, in welchen R⁷ Wasserstoff, (C₁-C₄)-Alkyl, (C₁-C₄)-Alkylcarbonyl, oder (C₁-C₄)-Alkoxycarbonyl bedeutet.

9. Verbindungen der Formel (I) gemäß einem der vorherigen Ansprüche, in welchen X CH oder CF bedeutet.

10. Verbindungen der Formel (I) gemäß einem der vorherigen Ansprüche, in welchen n 0 oder 1 ist.

11. Herbizide Mittel, **gekennzeichnet durch** einen herbizid wirksamen Gehalt an mindestens einer Verbindung der Formel (I) gemäß einem der Ansprüche 1 bis 10.

12. Herbizide Mittel gemäß Anspruch 11 in Mischung mit Formulierungshilfsmitteln.

13. Herbizide Mittel gemäß einem der Ansprüch 11 oder 12, enthaltend mindestens einen weiteren pestizid wirksame Stoffen aus der Gruppe Insektizide, Akarizide, Herbizide, Fungizide, Safenern und Wachstumsregulatoren.

14. Herbizide Mittel gemäß Anspruch 11 oder 12 enthaltend einen Safener.

15. Herbizide Mittel gemäß Anspruch 14 enthaltend ein weiteres Herbizid.

16. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 10 oder eines herbiziden Mittels nach einem der Ansprüche 11 bis 15 auf die Pflanzen oder auf den Ort des unerwünschten Pflanzenwachstums appliziert.

17. Verwendung von Verbindungen der Formel (I) gemäß einem der Ansprüche 1 bis 9 oder eines herbiziden Mittels nach einem der Ansprüche 11 bis 15 zur Bekämpfung unerwünschter Pflanzen.

18. Verwendung nach Anspruch 17, **dadurch gekennzeichnet, daß** die Verbindungen der Formel (I) zur Bekämpfung unerwünschter Pflanzen in Kulturen von Nutzpflanzen eingesetzt werden.

19. Verwendung nach Anspruch 18, **dadurch gekennzeichnet, daß** die Nutzpflanzen transgene Nutzpflanzen sind.
